(19) Europäisches Patentamt
European Patent Office
Office européen des brevets

(11) EP 1 853 355 B1

(12) **EUROPEAN PATENT SPECIFICATION**

(45) Date of publication and mention of the grant of the patent:
**19.05.2010 Bulletin 2010/20**

(21) Application number: **06737522.0**

(22) Date of filing: **03.03.2006**

(51) Int Cl.:
***A61Q 19/10*** *(2006.01)* ***A61K 8/37*** *(2006.01)*
***A61K 8/92*** *(2006.01)* ***A61K 8/31*** *(2006.01)*

(86) International application number:
**PCT/US2006/008356**

(87) International publication number:
**WO 2006/094309 (08.09.2006 Gazette 2006/36)**

(54) **RINSE-OFF OR WIPE-OFF SKIN CLEANSING COMPOSITIONS**

ABWASCHBARE ODER ABWISCHBARE HAUTREINIGUNGSZUSAMMENSETZUNGEN

COMPOSITIONS DE RINÇAGE OU DE NETTOYAGE DE LA PEAU

(84) Designated Contracting States:
**AT BE BG CH CY CZ DE DK EE ES FI FR GB GR HU IE IS IT LI LT LU LV MC NL PL PT RO SE SI SK TR**

(30) Priority: **04.03.2005 US 658687 P**
**20.07.2005 US 700960 P**

(43) Date of publication of application:
**14.11.2007 Bulletin 2007/46**

(73) Proprietor: **The Procter and Gamble Company**
**Cincinnati, Ohio 45202 (US)**

(72) Inventors:
- **CLAPP, Mannie, Lee**
  **Mason, Ohio 45040 (US)**
- **TAYLOR, Rebecca, Ann**
  **Cincinnati, Ohio 45241 (US)**
- **COFFINDAFFER, Timothy, Woodrow**
  **Maineville, OH 45039 (US)**
- **MCHUGH, Colin, Michael**
  **Mason, Ohio 45040 (US)**
- **KUHLMAN, Dennis, Eugene**
  **Middletown, Ohio 45044 (US)**
- **SEARS, Daniel, Burton**
  **Hamilton, Ohio 45011 (US)**
- **KYTE, Kenneth, Eugene, III**
  **Lebanon, Ohio 45036 (US)**

(74) Representative: **Wilding, Richard Alan**
**Procter & Gamble**
**Technical Centres Ltd**
**Rusham Park**
**Whitehall Lane**
**Egham, Surrey TW20 9NW (GB)**

(56) References cited:
**EP-A- 1 405 634** **WO-A-95/26710**
**WO-A-96/17592** **WO-A-98/30193**
**WO-A-03/028680** **WO-A-03/066016**
**WO-A-2004/065536** **WO-A-2006/021350**
**DD-A1- 150 759** **DE-A1-102004 045 253**
**FR-A- 2 843 540** **FR-A- 2 843 541**
**FR-A- 2 863 873** **US-A1- 2003 180 243**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 853 355 B1

## Description

FIELD OF THE INVENTION

The present invention relates to the field of personal care compositions for improving appearance and feel of keratinous surfaces. More specifically, the present invention relates to rinsable personal care compositions that provide excellent skin appearance, skin moisturization and conditioning, and/or skin cleansing.

BACKGROUND OF THE INVENTION

Personal care compositions are well known and widely used. These compositions have long been employed to cleanse and moisturize skin, deliver actives, hide imperfections and to reduce the oiliness/shine associated with sebum.

While the compositions and disclosures of the prior art provide useful advances in the art of personal care compositions, there remains the need for compositions that deliver immediate improvements in appearance and skin feel that will effectively deposit on all parts of the body. The compositions also need to be easy to apply and should not have a greasy or tacky feeling after application. It is further desirable for the compositions to deliver the above skin conditioning and appearance benefits via an application and removal process, such as in-the-shower or in-the-bath lotion, or a keratin-cleansing composition. Unfortunately for these applications, the moisturizers contained in the compositions are often readily rinsed or otherwise removed from the skin; this is particularly true when surfactant is also present. In recent publications, compositions containing structured lipids have been taught to provide enhanced deposition of moisturizers to the skin. It has been recognized that there is increased tackiness of the coating experienced by users when compositions contain more structured lipids and/or lipids with a higher modulus. It is now believed that utilizing a combination of high modulus lipids and esters in compositions, that the deposition of moisturizers can be maintained, while the tackiness of the coating can be substantially reduced upon rinsing, wiping or otherwise removing the product from the keratin surface. Therefore, it is desirable to provide a topical, rinseable and/or removable compositions that comprise a select level of skin benefit agent which include a high modulus lipids blended with esters to provide a unique level of skin moisturization without tackiness or greasiness across all skin types.

SUMMARY OF THE INVENTION

The present invention relates to a rinsable personal care composition that comprise

a) from 0, 1 % to 50% by weight of said composition, of surfactant of which from 0,01 to 4% by weight of the composition is non-ionic surfactant;
b) from 18% to 70% by weight of said composition, of a skin benefit agent; said skin benefit agent comprising:

a high modulus lipid, having a lipid modulus from 50 to 5000 Pa; and
an ester, said ester having less than 30 carbon atoms; and wherein the ratio by weight of high modulus lipid to ester is in the range from 30:1 to 1:15; and

c) at least 20 % by weight of said composition, of water but wherein the total amount of water and hydroxyl containing solvents in the composition is less than 80% by weight of the composition.

The present invention also relates to a process for moisturizing the skin that comprises the steps of: (a) cleansing the skin; (b) applying to the skin a composition comprising

a) from 0, 1 % to 50% by weight of said composition, of surfactant of which from 0,01 to 4% by weight of the composition is non-ionic surfactant;
b) from 18% to 70% by weight of said composition, of a skin benefit agent; said skin benefit agent comprising:

a high modulus lipid, having a lipid modulus from 50 to 5000 Pa; and
an ester, said ester having less than 30 carbon atoms; and wherein the ratio by weight of high modulus lipid to ester is in the range from 30:1 to 1:15; and

c) at least 20 % by weight of said composition, of water but wherein the total amount of water and hydroxyl containing solvents in the composition is less than 80% by weight of the composition.

and (c) rinsing said composition.

The present invention also relates to a process for removing make-up that comprises the steps of: (a) applying the skin a composition comprising

a) from 0, 1 % to 50% by weight of said composition, of surfactant of which from 0,01 to 4% by weight of the composition is non-ionic surfactant;
b) from 18% to 70% by weight of said composition, of a skin benefit agent; said skin benefit agent comprising:

a high modulus lipid, having a lipid modulus from 50 to 5000 Pa; and
an ester, said ester having less than 30 carbon atoms; and wherein the ratio by weight of high modulus lipid to ester is in the range from 30:1 to 1:15; and

c) at least 20 % by weight of said composition, of water but wherein the total amount of water and hydroxyl containing solvents in the composition is less than 80% by weight of the composition.

and (b) rinsing or wiping away said composition.

DETAILED DESCRIPTION OF THE INVENTION

All percentages and ratios used herein are by weight of the total composition and all measurements made are at 25°C, unless otherwise designated.

The term "**amphoteric surfactant,**" as used herein, is also intended to encompass zwitterionic surfactants, which are well known to formulators skilled in the art as a subset of amphoteric surfactants.

The term "**dermatologically-acceptable**," as used herein, means that the compositions or components thereof so described are suitable for use in contact with human skin without undue toxicity, incompatibility, instability, allergic response, and the like.

The term "**diameter**" as used herein, means the largest distance across the major axis of the particulate material. Diameter can be determined by any suitable method known in the art, such as particle size analyzer Mastersizer 2000 manufactured by Malvern Instruments.

As used herein "**high modulus lipid**," means a lipid that has a lipid modulus of at least 50 Pa, preferably at least 75 Pa, 100 Pa, more preferably at least 200 Pa, even more preferably more than 300 Pa, even more preferably greater than 400 Pa, more preferably greater than 600 Pa as, and most preferably 700 Pa determined using the Lipid Modulus Test described in the Analytical Methods section below. The high modulus lipid should have a lipid modulus of no more than 5000 Pa, preferably no more than 4000 Pa, preferably no more than 3000 Pa , even more preferably no more than 2000 Pa and most preferably less than 1000 Pa.

The term "**hydrophobically modified interference pigment**" or "**HMIP**", as used herein, means a portion of the interference pigment surface has been coated with a hydrophobic material. Hydrophobically modified interference pigments that are suitable for use in the compositions of the present invention are those disclosed in US Patent Application Publication No. 2004/0223929 A1, published November 11, 2004.

The term "**interference pigment**", as used herein, means a pigment with pearl gloss prepared by coating the surface of a particle substrate material (generally platelet in shape) with a thin film. The thin film is a transparent or semitransparent material having a high refractive index. The higher refractive index material shows a pearl gloss resulting from mutual interfering action between reflection and incident light from the platelet substrate/coating layer interface and reflection of incident light from the surface of the coating layer.

The term "**lipid modulus**" as used herein is a term often used to describe deformations of solid, and in this case its use reflects the fact that the structurants add a solid-like nature to the lipid. This can aid in effectively balancing deposition, retention and spreading (feel) of the lipid blend on the skin. Lipid modulus is as determined using the Lipid Modulus Test described below in the Analytical Methods section below.

As used herein, "non-platelet particle" refers to any shape of a particle in the personal care composition differing from the platelet shape including, but not limited to, configurations such as spherical, cylindrical configuration, rectangular, triangular, trapezoidal, semi-circular, hourglass, or irregular shapes. The non-platelet particles may have an aspect ratio define by A/B = or >0.20, the length A and the width being B, more preferably greater than 0.25, even more preferably greater than 0.3. Non-platelet particles and hydrophobically modified non-platelet particles that are suitable for use in the present application are disclosed in US Patent Application Serial No. 11/057,957, filed on February 15, 2005 by Taylor et al., titled "Personal Care Compositions Containing Hydrophobically Modified Non-Platelet Particles."

The term "**personal care composition**" as used herein, unless otherwise specified, refers to the compositions of the present invention, wherein the compositions are intended for topical application to the skin or hair.

The term "rinsable **composition**" as used herein, means a composition designed to be rinsed off by a liquid such as water. After the composition is rinsed off, benefit agents remain on the skin.

The term "**safe and effective amount**" as used herein means an amount of a compound, component, or composition sufficient to significantly induce a positive benefit, preferably a positive skin appearance or feel benefit, including independently the benefits disclosed herein, but low enough to avoid serious side effects, i.e., to provide a reasonable benefit to risk ratio, within the scope of sound medical judgment.

A "**skin compatible oil**" as defined herein, is an oil that is liquid or semi-solid at the temperature at which bathing is carried out that is deemed safe for use in cosmetics being either inert to the skin or actually beneficial.

The term "**topical application**", as used herein, means to apply or spread the compositions of the present invention onto the surface of the skin.

Active and other ingredients useful herein may be categorized or described herein by their cosmetic and/or therapeutic benefit or their postulated mode of action. However, it is to be understood that the active and other ingredients useful herein can in some instances provide more than one cosmetic and/or therapeutic benefit or operate via more than one mode of action. Therefore, classifications herein are made for the sake of convenience and are not intended to limit an ingredient to the particularly stated application or applications listed.

### A. Surfactant

The rinsable personal care composition of the present invention comprises from 0,1% to 50% by weight of said composition of surfactant of which from 0,01 to 4% by weight of the composition as non-ionic surfactant . A wide variety of surfactants can be useful herein, both for emulsification of the dispersed phase as well as to provide acceptable spreading and in use properties for non-lathering systems. For cleansing applications, the surfactant phase also serves to clean the skin and provide a desirable experience for the user. The composition contains no more than 50 weight percent of a surfactant, more preferably no more than 30 weight percent, still more preferably no more than 15 weight percent, and even more preferably no more than 5 weight percent of a surfactant. The composition preferably contains at least 5 weight percent of a surfactant, more preferably at least 3 weight percent, still more preferably at least Metal 1 weight percent, and even more preferably at least 0.1 weight percent of a surfactant.

Preferable surfactants include those selected from the group consisting of anionic surfactants, nonionic surfactants, amphoteric surfactants, cationic surfactants, non-lathering surfactants, emulsifiers and mixtures thereof. Non-limiting' examples of surfactants useful in the compositions of the present invention are disclosed in U.S. Patent No. 6,280,757, to McAtee et al., issued Aug. 28, 2001.

### i. Anionic Surfactants

Nonlimiting examples of anionic surfactants useful in the compositions of the present invention are disclosed in McCutcheon's, Detergents and Emulsifiers, North American edition (1986), published by allured Publishing Corporation; McCutcheon's, Functional Materials, North American Edition (1992); and U.S. Patent No. 3,929,678, to Laughlin et al., issued Dec. 30, 1975.

A wide variety of anionic surfactants are useful herein. Nonlimiting examples of anionic surfactants include those selected from the group consisting of sarcosinates, sulfates, isethionates, taurates, phosphates, lactylates, glutamates, and mixtures thereof. Amongst the isethionates, the alkoyl isethionates are preferred, and amongst the sulfates, the alkyl and alkyl ether sulfates are preferred.

Other anionic materials useful herein are fatty acid soaps (i.e., alkali metal salts, e.g., sodium or potassium salts) typically having from a fatty acid having 8 to 24 carbon atoms, or preferably from 10 to 20 carbon atoms. These fatty acids used in making the soaps can be obtained from natural sources such as, for instance, plant or animal-derived glycerides (e.g., palm oil, coconut oil, soybean oil, castor oil, tallow, lard, etc.) The fatty acids can also be synthetically prepared. Soaps and their preparation are described in detail in U.S. Patent. No. 4,557,853.

Other anionic materials include phosphates such as monoalkyl, dialkyl, and trialkylphosphate salts. Nonlimiting examples of preferred anionic lathering surfactants useful herein include those selected from the group consisting of sodium lauryl sulfate, ammonium lauryl sulfate, ammonium laureth sulfate, sodium laureth sulfate, sodium trideceth sulfate, ammonium cetyl sulfate, sodium cetyl sulfate, ammonium cocoyl isethionate, sodium lauroyl isethionate, sodium lauroyl lactylate, triethanolamine lauroyl lactylate, sodium caproyl lactylate, sodium lauroyl sarcosinate, sodium myristoyl sarcosinate, sodium cocoyl sarcosinate, sodium lauroyl methyl taurate, sodium cocoyl methyl taurate, sodium lauroyl glutamate, sodium myristoyl glutamate, and sodium cocoyl glutamate and mixtures thereof.

Especially preferred for use herein is ammonium lauryl sulfate, ammonium laureth sulfate, sodium lauroyl sarcosinate, sodium cocoyl sarcosinate, sodium myristoyl sarcosinate, sodium lauroyl lactylate, and triethanolamine lauroyl lactylate.

### ii. Nonionic Surfactants

Nonionic surfactants are incorporated in the present compositions to enhance the rinseability of the present compositions from the skin. Nonionic surfactants are typically incorporated in the present compositions at a level of from 0.01% to 4%,preferably from 0.1% to 3%, and more preferably from 0.2% to 2%, by weight of the composition.

Among the nonionic surfactants that are useful herein are those that can be broadly defined as condensation products of long chain alcohols, e.g. $C_{8-30}$ alcohols, with sugar or starch polymers, i.e., glycosides. These compounds can be represented by the formula $(S)_n$-O-R wherein S is a sugar moiety such as glucose, sucrose, fructose, mannose, or galactose, n is an integer of from 1 to 1000, and R is a $C_{8-30}$ alkyl group. Examples of long chain alcohols from which the alkyl group can be derived include decyl alcohol, cetyl alcohol, stearyl alcohol, lauryl alcohol, myristyl alcohol, oleyl alcohol, and the like. Preferred examples of these surfactants include those wherein S is a glucose moiety, R is a $C_{8-20}$ alkyl group, and n is an integer of from 1 to 9. Commercially available examples of these surfactants include decyl polyglucoside and lauryl polyglucoside commercially-available from Cognis.

Other useful nonionic surfactants included the condensation products of sorbitol with a fatty acid. Non-limiting examples include the Tweens, Spans, and the Polysorbates.

Other useful nonionic surfactants include the condensation products of alkylene oxides with fatty acids (i.e. alkylene oxide esters of fatty acids). These materials have the general formula $RCO(X)_nOH$ wherein R is a C10-30 alkyl group, X is $-OCH_2CH_2-$(i.e. derived from ethylene glycol or oxide) or $-OCH_2CHCH_3-$ (i.e. derived from propylene glycol or oxide), and n is an integer from 6 to 100. Non-limiting examples of these alkylene oxide derived nonionic surfactants include ceteth-6, ceteth-10, ceteth-12, ceteareth-6, ceteareth-10, ceteareth-12, steareth-6, steareth-10, steareth-12, PEG-6 stearate, PEG-10 stearate, PEG-12 stearate, PEG-20 glyceryl stearate, PEG-80 glyceryl tallowate, PPG-10 glyceryl stearate, PEG-30 glyceryl cocoate, PEG-80 glyceryl cocoate, PEG-200 glyceryl tallowate, PEG-8 dilaurate, PEG-10 distearate, and mixtures thereof.

Still other useful nonionic surfactants include polyhydroxy fatty acid amide surfactants. A preferred polyhydroxy fatty acid amide surfactant is coconut alkyl N-methyl glucoside amide. Processes for making compositions containing polyhydroxy fatty acid amides are disclosed, for example, in G. B. Patent Specification 809,060, published Feb. 18, 1959, by Thomas Hedley & Co., Ltd.; U.S. Pat. No. 2,965,576, to E. R. Wilson, issued Dec. 20, 1960; U.S. Pat. No. 2,703,798, to A. M. Schwartz, issued Mar. 8, 1955; and U.S. Pat. No. 1,985,424, to Piggott, issued Dec. 25, 1934.

Non-limiting examples of suitable nonionic surfactants include: polyethylene glycol 20 sorbitan monolaurate (Polysorbate 20), polyethylene glycol 5 soya sterol, steareth-20, steareth-21, ceteareth-20, ceteareth-12, PPG-2 methyl glucose ether distearate, ceteth-10, Polysorbate 80, cetyl phosphate, potassium cetyl phosphate, diethanolamine cetyl phosphate, Polysorbate 60, glyceryl stearate, PEG-100 stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, diethanolamine cetyl phosphate, glyceryl stearate, PEG-100 stearate, and mixtures thereof. Preferred nonionic surfactants are those selected from the group consisting of steareth-21, ceteareth-20, ceteareth-12, Tween-60, Tween-80, sucrose cocoate, steareth-100, PEG-100 stearate, PEG-1000 stearate, and mixtures thereof.

Nonionic surfactants can be useful in facilitating the rinse properties of a composition comprising a gel network. Preferred nonionic surfactants will typically have an average HLB value of at least 8, preferably at least 9, more preferably at least 10, more preferably at least 11, and more preferably at least 12. Additionally, preferred nonionic surfactants will have an average HLB value of no greater than 20, preferably no greater than 18, more preferably no greater than 16, and more preferably no greater than 14.

Additionally, preferred nonionic surfactants for facilitating rinse properties of the present compositions, especially those comprising a gel network, have alkyl chain lengths of from $C_8$ to $C_{16}$, preferably from $C_{10}$ to $C_{14}$, and more preferably from $C_{10}$ to $C_{12}$. Non-limiting examples of such nonionic surfactants include $C_8$-$C_{14}$ glucose amides, $C_8$-$C_{14}$ alkyl polyglucosides, $C_8$-$C_{14}$ alkyl glucosides, and $C_8$-$C_{14}$ alkyl ethoxylates.

### iii. Amphoteric Surfactants

A wide variety of amphoteric lathering surfactants can be used in the compositions of the present invention. Particularly useful are those which are broadly described as derivatives of aliphatic secondary and tertiary amines, preferably wherein the nitrogen is in a cationic state, in which the aliphatic radicals can be straight or branched chain and wherein one of the radicals contains an ionizable water solubilizing group, e.g., carboxy, sulfonate, sulfate, phosphate, or phosphonate.

Nonlimiting examples of amphoteric surfactants useful in the compositions of the present invention are disclosed in McCutcheon's, Detergents and Emulsifiers, North American edition (1986), published by allured Publishing Corporation; and McCutcheon's, Functional Materials, North American Edition (1992).

Nonlimiting examples zwitterionic surfactants are those selected from the group consisting of betaines, sultaines, hydroxysultaines, alkyliminoacetates, imninodialkanoates, aminoalkanoates, and mixtures thereof.

### iv. Cationic Surfactants

Nonlimiting examples of cationic surfactants useful herein include cationic ammonium salts such as those having the formula:

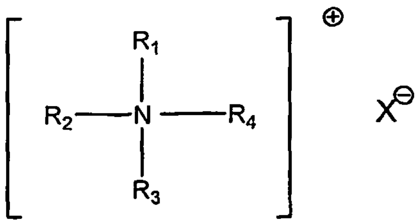

wherein $R_1$, is selected from an alkyl group having from 12 to 22 carbon atoms, or from aryl or alkaryl groups having from 12 to 22 carbon atoms; $R_2$, $R_3$, and $R_4$ are independently selected from hydrogen, an alkyl group having from 1 to 22 carbon atoms, or aromatic, aryl or alkaryl groups having from 6 to 22 carbon atoms; and X is an anion selected from chloride, bromide, iodide, acetate, phosphate, nitrate, sulfate, methyl sulfate, ethyl sulfate, tosylate, lactate, citrate, glycolate, and mixtures thereof. Additionally, the alkyl groups can also contain either linkages, or hydroxy or amino group substituents (e.g., the alkyl groups can contain polyethylene glycol and polyproylene glycol moieties). Preferred examples of cationic surfactants include distearyl dimethyl ammonium chloride and/or dipalmityl dimethyl ammonium chloride.

Other suitable cationic surfactants include protonated amines. The cationic protonated amines can be primary, secondary, or tertiary amines (preferably secondary or tertiary), depending upon the particular species and the selected pH of the present composition. Non-limiting examples of cationic protonated amines include stearamidopropyl dimethylamine, cocamidopropyl dimethylamine, lauramidopropyl dimethylamine, oleamidopropyl dimethylamine, palmitamidopropyl dimethylamine, and mixtures thereof.

### B. Aqueous Phase Hydrophobic Structuring Agent

The compositions of the present invention optionally comprise no more than 20%, preferably no more than 10%, and more preferably no more than 7.5%, by weight of the composition, of an aqueous phase hydrophobic structuring agent, preferably selected from the group consisting of saturated $C_{16}$ to $C_{30}$ fatty alcohols, saturated $C_{16}$ to $C_{30}$ fatty alcohols containing from 1 to about 5 moles of ethylene oxide, saturated $C_{16}$ to $C_{30}$ diols, saturated $C_{16}$ to $C_{30}$ monoglycerol ethers, saturated $C_{16}$ to $C_{30}$ hydroxy fatty acids, and mixtures thereof, having a melting point of at least 40°C. The present invention optionally, but preferably, comprises at least 0.5%, more preferably at least 1%, and even more preferably at least 2%, by weight of the composition, of an aqueous phase hydrophobic structuring agent, preferably selected from the group consisting of saturated $C_{16}$ to $C_{30}$ fatty alcohols, saturated $C_{16}$ to $C_{30}$ fatty alcohols containing from about 1 to about 5 moles of ethylene oxide, saturated $C_{16}$ to $C_{30}$ diols, saturated $C_{16}$ to $C_{30}$ monoglycerol ethers, saturated $C_{16}$ to $C_{30}$ hydroxy fatty acids, and mixtures thereof, having a melting point of at least 40°C. These structuring agents can be useful to assist in the formation of the rheological characteristic of the composition which can contribute to the stability of the composition of the present invention. In particular, the aqueous phase hydrophobic structuring agents tend to assist in the formation of the liquid crystalline gel network structures in the present compositions. Indeed, the present compositions preferably comprise a gel network, and preferably a nonionic gel network. The "gel network" of the present invention typically comprises an aqueous phase hydrophobic structuring agent and a surfactant. A gel network in the present compositions tends to provide a rich creamy feel and to allow good application of the product without causing damage to the skin.

Suitable hydrophobic structuring agents of the present invention are selected from the group consisting of stearyl alcohol, cetyl alcohol, cetearyl alcohol, myristyl alcohol, arachidic alcohol, behenyl alcohol, stearic acid, palmitic acid, the polyethylene glycol ether of stearyl alcohol having an average of 1 to 5 ethylene oxide units, the polyethylene glycol ether of cetyl alcohol having an average of 1 to 5 ethylene oxide units, emulsifying waxes (e.g. POLAWAX® NF available from Croda), and mixtures thereof. Non-limiting examples include PROLIPID 141 (glyceryl stearate, behenyl alcohol, palmitic acid, stearic acid, lecithin, lauryl alcohol, myristyl alcohol and cetyl alcohol) and PROLIPID 151 (Glyceryl stearate, cetearyl alcohol, stearic acid, 1-propanamium, 3-amino-N-(2-(hydroxyethyl)-N-N-Dimethyl,N-C(16-18) Acyl Derivatives, Chlorides) from ISP; POLAWAX NF (Emulsifying wax NF), INCROQUAT BEHENYL TMS (behentrimonium sulfate and cetearyl alcohol) from Croda; and EMULLIUM DELTA (cetyl alcohol, glyceryl stearate, peg-75 stearate, ceteth-20 and steareth-20) from Gattefosse. Preferred structuring agents are selected from the group consisting of stearyl alcohol, cetyl alcohol, behenyl alcohol, steareth-2, and mixtures thereof. Such hydrophobic structuring agents

can be provided as mixtures with other hydrophobic structuring agents and/or surfactants.

### C. Skin Benefit Agent

The compositions of the present invention comprise 18 % to 70% weight percent of a skin benefit agent that comprises a high modulus lipid and an ester. The high modulus lipid comprises a skin compatible oil, gel, or wax, or mixtures thereof. By definition, the skin benefit agent will have negligible solubility in any aqueous phase and may be present as discrete particles in the composition. The skin benefit agent preferably comprises no more than 70 weight percent, still more preferably no more than 60 weight percent, and still more preferably no more than 50 weight percent of the composition. The skin benefit agent comprises at least 18 weight percent of the composition.

The shear index is a measure of how shear thinning the skin benefit agent is as described in the Lipid Rheology Test described herein. It is preferred that the skin compatible oil be shear thinning either by virtue of its composition or the structurants that may be added. Preferably, the shear index of the skin benefit agent will preferably be less than 0.9, more preferably less than 0.75, even more preferably less than 0.6, even more preferably less than 0.5, and still more preferably less than 0.4.

The high modulus lipid may comprise oils selected from the group consisting of triglycerides, hydrocarbon oils, polyesters, silicone oils and mixtures thereof.

One class of useful oils is the triglycerides and modified triglycerides. These include vegetable oils such as jojoba, soybean, canola, sunflower, safflower, rice bran, avocado, almond, olive, sesame, persic, castor, coconut, and mink oils. Synthetic triglycerides can also be employed. Modified triglycerides include materials such as ethoxylated and maleated triglyceride derivatives provided they are liquids. Proprietary ester blends such as those sold by Finetex as Finsolv are also suitable, as is ethylhexanoic acid glyceride.

Another type of oil is liquid polyester formed from the reaction of a dicarboxylic acid and a diol. Examples of polyesters suitable for the present invention are the polyesters marketed by ExxonMobil under the trade name PURESYN ESTER®.

A second class of skin compatible oils suitable for the present invention is liquid and semi-solid hydrocarbons. These include linear and branched oils such as liquid paraffin, squalene, squalane, mineral oil, low viscosity synthetic hydrocarbons such as polyalphaolefin sold by ExxonMobil under the trade name of PURESYN PAO and polybutene under the trade name PANALANE or INDOPOL. Light (low viscosity) highly branched hydrocarbon oils are also suitable.

Petrolatum is a unique hydrocarbon material and a useful component of the present invention. Its semi-solid nature can be controlled both in production and by the formulator through blending with other oils.

A third class of useful skin compatible oils is silicone based. They include linear and cyclic polydimethyl siloxane, organo functional silicones (alkyl and alkyl aryl), and amino silicones.

The high modulus lipid may optionally comprise a structurant. The structurant can provide the high modulus lipid with the correct rheological properties in particular the appropriate modulus. The amount of structurant required to produce this viscosity will vary depending on the oil and the structurant, but in general, the structurant will preferably be less than 75 weight percent of the dispersed oil phase, more preferably less than 60 weight percent, and still more preferably less than 50 weight percent of the dispersed oil phase.

Structurants meeting the above requirements with the selected skin compatible oil can form 3-dimensional network to build up the viscosity of the selected oils. It has been found that such structured oil phases, i.e., built with the 3-dimensional network, are extremely desirable for use as wet-skin treatment compositions used in bathing. These structured oils can deposit and be retained very effectively on wet skin and retained after rinsing and drying to provide long-lasting after wash skin benefit without causing a too oily/greasy wet and dry feel. It is believed that the highly desirable in-use and after-use properties of such structured oils are due to their shear thinning rheological properties and the weak structure of the network. Due to its high low-shear viscosity, the 3-dimensional network structured oil can stick and retain well on the skin during application of the skin conditioner. After being deposited on the skin, the network yields easily during rubbing due to the weak structuring of the crystal network and its lower high-shear viscosity.

The structurant can be either an organic or inorganic structurant. Examples of organic structurants suitable for the invention can be selected from the group consisting of solid fatty acid esters, natural or modified fats, fatty acid, fatty amine, fatty alcohol, natural and synthetic waxes, petrolatum, block copolymers, and mixtures thereof. Suitable block polymers for this application can be those sold under the name KRATON by Shell. Inorganic structuring agents can be selected from the group consisting of hydrophobically modified silica, hydrophobically modified clay and mixtures thereof. Non-limiting examples of inorganic structurants are BENTONE 27V, BENTONE 38V or BENTONE GEL MIO V from Rheox; and CAB-O-SIL TS720 or CAB-O-SIL M5 from Cabot Corporation.

The structurant may be crystalline may be a natural or synthetic crystalline wax. Mineral, animal or plant (vegetable) waxes are all described as natural waxes. Synthetic waxes are described as those waxes that have been synthetically polymerized from raw materials or chemically modified natural waxes.

Among the natural crystalline waxes which may be used are petroleum based waxes such as paraffins and

microcrystalline wax. Molecular weights of paraffin waxes generally range from 360 to 420 (26 to 30 carbon atoms), although versions with longer chains (molecular weights up to 600) are available. Typical melting points are 126-134°F. (52-57°C), the high molecular weight versions have melting points near 170°F (77°C). Paraffin waxes are brittle and the addition of oil weakens the structure (lowers the tensile strength).

Microcrystalline waxes (MC) melting points are 145 to 195°F (63-91°C). The crystals of MC wax are small and irregular and consist of several types: plates, malcrystalline and needle. Animal waxes can be obtained from such things as bees, insects or whales. These waxes include but are not limited to beeswax, Chinese wax, shellac wax, spermaceti and wool wax. Plant waxes can be derived from beans, leaves and berries. Plant or vegetable waxes can include bayberry, candelilla, carnauba, cotton, esparto, fir, Japan, ouricury, palm, rice-oil, sugar cane, ucuhuba and cocoa butter.

Among synthetic crystalline waxes which may be used are crystalline polymers such as polyethylene, Fischer-Tropsch waxes such as polymethylene, chemically modified waxes, polymerized alpha olefins and synthetic animal waxes. For example, siliconyl beeswax may be used which is beeswax that has been chemically modified.

Another structurant that may be optionally used in the present invention is the microcrystalline wax petrolatum (also known as petrolatum or mineral jelly), which typically comprises 90% by wt. of a natural mixture of microcrystalline waxes plus minor amounts of other impurities

In addition, structurant may be a natural or synthetic hydrogenated oils or fats. In addition some fatty acids and fatty alcohols can be used as structurant as well as salts of fatty acids, hydroxy fatty acids and fatty acid esters.

Hydrogenated oils can be hydrogenated vegetable oils, hydrogenated coconut oil, hydrogenated palm kernel oil, hydrogenated rapeseed oil, castorwax and many others.

Along with size and shape, a high concentration of particles is required so that the crystals interact in the dispersion. Above a certain critical volume fraction of crystals, these interactions will lead to a buildup of a network that extends throughout the whole volume. The crystal network creates a solid-like material having viscoelastic (solid-like and liquid-like) properties.

Crystalline long chain fatty acids and long chain fatty alcohols can also be used to structure benefit agents. Examples of fatty acids are myristic acid, palmitic acid, stearic acid, arachidic acid and behenic acid. Examples of fatty alcohols are palmityl alcohol, stearyl alcohol, arachyl alcohol and behenyl alcohol. Some crystalline fatty acid esters and glyceride esters will also provide structuring benefit.

In addition, the crystalline materials can be combined with other structuring materials such as natural and synthetic waxes to form composite networks to structure benefit agents.

The skin benefit agent of the present invention comprises an ester,said ester having less than 30 carbon atoms. Esters may serve to enhance the spreadability of the oil and to reduce the tack typically associated with structured oils of high modulus lipids. The esters can be premixed with the structured oil phase, or they can be added separately while the product is warm, or when the product is cooling.

The esters of the present invention may include ester oils and as the name implies, ester oils comprise at least one ester group in the molecule. One type of common ester oil useful in the present invention are the fatty acid mono and polyesters such as cetyl octanoate, octyl isonanoanoate, myristyl lactate, cetyl lactate, isopropyl myristate, myristyl myristate, diisopropyl sebacate, diisotearyl malate, isostearyl neopentanoate, isopropyl palmitate, isopropyl adipate, butyl stearate, decyl oleate, cholesterol isostearate, glycerol monostearate, glycerol distearate, glycerol tristearate, alkyl lactate, alkyl citrate and alkyl tartrate; sucrose ester and polyesters, sorbitol ester, and the like.

The ester may be selected from the group consisting of a di-ester, tri-ester, tetra-ester, a branched ester, a dimer and mixtures thereof. Non-limiting examples of diesters may include diisopropyl adipate or diisopropyl sebacate. Non-limiting examples of branched ester may include ethylhexyl isononanoate. Non-limiting examples of dimers may include diisopropyl dimer dilinoleate. Non-limiting examples of tetraesters include the pentaerythritol esters

The esters will preferably have a viscosity below 100 mPas, even more preferably below 75 mPas, even more preferably below 50 mPas, and most preferably below 40 mPas.

The ester's individual fatty groups will preferably comprise no more than 20 carbons, even more preferably no more than 17 carbons, even more preferably, no more than 14, and most preferably no more than 12. The total number of carbon atoms in the ester will be less than 30, even more preferably less than 25, even more preferably less than 23

Preferably the ratio of high modulus lipid to ester can comprise no more than 30:1, even more preferably no more than 20:1, even more preferably no more than 10:1, and most preferably no more than 2:1 Preferably the ratio of high modulus lipid to ester can comprise at least 1:15, even more preferably more than 1:10, even more preferably no more than 1:8, even more preferably no more than 1:4, even more preferably no more than 1:2, and most preferably no more than 1:1.

Esters can be added to the product in a variety of methods, as discussed in the Examples section below.

### D. Auxiliary Skin Benefit Agents

The personal care compositions of the present invention may optionally further comprise an auxiliary skin

benefit agent suitable for use on the skin, and which is otherwise compatible with the other selected ingredients in the composition. The skin benefit agent can be blended with the oils previously described; in this case the oil may function as a carrier for the auxiliary skin benefit agent. The auxiliary skin benefit agent may also be included separately from the oil phase.

Non-limiting examples of auxiliary kin benefit agents suitable for use herein are described in The CTFA Cosmetic Ingredient Handbook, Second Edition (1992), which includes a wide variety of cosmetic and pharmaceutical ingredients commonly used in the skin care industry, and which are suitable for use in the compositions of the present invention. Non-limiting examples of such auxiliary skin benefit agents include abrasives, absorbents, aesthetic components such as fragrances, pigments, colorings/colorants, essential oils, skin sensates, astringents, etc. (e.g., clove oil, menthol, camphor, eucalyptus oil, eugenol, menthyl lactate, witch hazel distillate), anti-acne agents, anti-caking agents, antimicrobial agents (e.g., iodopropyl butylcarbamate), antioxidants, colorants, cosmetic astringents, cosmetic biocides, drug astringents, external analgesics, opacifying agents, pH adjusters, skin bleaching and lightening agents (e.g., hydroquinone, kojic acid, ascorbic acid, magnesium ascorbyl phosphate, ascorbyl glucosamine), skin-conditioning and/or moisturizing agents, i.e. glycerine and other humectants, skin soothing and/or healing agents (e.g., panthenol and derivatives (e.g., ethyl panthenol), aloe vera, pantothenic acid and its derivatives, allantoin, bisabolol, and dipotassium glycyrrhizinate), retinoids, (e.g. retinol palmitate), tocopheryl nicotinate, skin treating agents, vitamins and derivatives thereof. In any embodiment of the present invention, however, the actives useful herein can be categorized by the benefit they provide or by their postulated mode of action. However, it is to be understood that the actives useful herein can in some instances provide more than one benefit or operate via more than one mode of action. Therefore, classifications herein are made for the sake of convenience and are not intended to limit the active to that particular application or applications listed. The skin benefit agents are furthered described hereinafter in details.

**i. Desquamation Actives**

The auxiliary skin benefit agent for use herein can include desquamation actives, preferred concentrations of which range from 0.1% to 10%, more preferably from 0.2% to 5%, even more preferably from 0.5% to 4%, by weight of the composition for non-surfactant containing actives and from 0.1% to 3%, more preferably from 0.2% to 3%, even more preferably from 0.5% to 3% for surfactant containing actives. Desquamation actives enhance the skin appearance benefits of the present invention. For example, the desquamation actives tend to improve the texture of the skin (e.g., smoothness). One desquamation system that is suitable for use herein contains sulfhydryl compounds and zwitterionic surfactants and is described in U.S. Patent No. 5,681,852, to Bissett.

Another desquamation system that is suitable for use herein contains salicylic acid and zwitterionic surfactants and is described in U.S. Patent No. 5,652,228 to Bissett.

**ii. Anti-Acne Actives**

The auxiliary skin benefit agent for use herein can also include anti-acne actives, preferred concentrations of which range from 0.01% to 50%, more preferably from 1% to 20%, by weight of the composition. Non-limiting examples of anti-acne actives suitable for use herein include resorcinol, sulfur, salicylic acid, benzoyl peroxide, erythromycin, zinc, and other similar materials.

Other non-limiting examples of suitable anti-acne actives for use herein are described in U. S. Patent No. 5,607,980, issued to McAtee et al, which description is incorporated herein by reference.

**iii. Anti-Wrinkle Actives/Anti-Atrophy Actives**

The auxiliary skin benefit agent for use herein can also include anti-wrinkle actives or anti-atrophy actives, including sulfur-containing D and L amino acids and their derivatives and salts, particularly the N-acetyl derivatives, a preferred example of which is N-acetyl-L-cysteine; thiols, e.g. ethane thiol; hydroxy acids (e.g., alpha-hydroxy acids such as lactic acid and glycolic acid or beta-hydroxy acids such as salicylic acid and salicylic acid derivatives such as the octanoyl derivative), phytic acid, lipoic acid; lysophosphatidic acid, and skin peel agents (e.g., phenol and the like). Also suitable is niacinamide.

Hydroxy acids as skin benefit agents herein include salicylic acid and salicylic acid derivatives, preferred concentrations of which range from 0.01% to 50%, more preferably from 0.1% to 10%, even more preferably from 0.5% to 2%, by weight of the composition.

Other non-limiting examples of suitable anti-wrinkle actives for use herein are described in U. S. Patent No. 6,217,888, issued to Oblong et al.

### iv. Anti-Oxidants/Radical Scavengers

The auxiliary skin benefit agent for use herein can also include anti-oxidants or radical scavengers, preferred concentrations of which range from 0.1% to 10%, more preferably from 1% to 5%, by weight of the composition.

Non-limiting examples of anti-oxidants or radical scavengers for use herein include ascorbic acid and its salts, ascorbyl esters of fatty acids, ascorbic acid derivatives (e.g., magnesium ascorbyl phosphate, sodium ascorbyl phosphate, ascorbyl sorbate), tocopherol, tocopherol acetate, other esters of tocopherol, butylated hydroxy benzoic acids and their salts, 6-hydroxy-2,5,7,8-tetramethylchroman-2-carboxylic acid (commercially available under the tradename Trolox®), gallic acid and its alkyl esters, especially propyl gallate, uric acid and its salts and alkyl esters, sorbic acid and its salts, lipoic acid, amines (e.g., N,N-diethylhydroxylamine, amino-guanidine), sulfhydryl compounds (e.g., glutathione), dihydroxy fumaric acid and its salts, lycine pidolate, arginine pilolate, nordihydroguaiaretic acid, bioflavonoids, curcumin, lysine, methionine, proline, superoxide dismutase, silymarin, tea extracts, grape skin/seed extracts, melanin, and rosemary extracts may be used.

### v. Chelators

The skin benefit agent for use herein can also include chelating agents. As used herein, the term "chelating agent" or "chelator" refers to those skin benefit agents capable of removing a metal ion from a system by forming a complex so that the metal ion cannot readily participate in or catalyze chemical reactions.

The chelating agents as skin benefit agents for use herein are preferably formulated at concentrations ranging from 0.1% to 10%, more preferably from 1% to 5%, by weight of the composition. Non-limiting examples of suitable chelating agents are described in U.S. Patent No. 5,487,884, issued 1/30/96 to Bissett et al.; International Publication No. 91/16035, Bush et al., published 10/31/95; and International Publication No. 91/16034, Bush et al., published 10/31/95.

Preferred chelating agents for use in the active phase of the compositions of the present invention include furildioxime, furilmonoxime, and derivatives thereof.

### vi. Flavonoids

The auxiliary skin benefit agent for use herein includes flavonoid compounds suitable for use on the hair or skin, preferred concentrations of which range from 0.01% to 20%, more preferably from 0.1% to 10%, more preferably from 0.5% to 5%, by weight of the composition.

Non-limiting examples of flavonoids compounds suitable for use as skin benefit agents include flavanones such as unsubstituted flavanones, mono-substituted flavanones, and mixtures thereof; chalcones selected from unsubstituted chalcones, mono-substituted chalcones, di-substituted chalcones, tri-substituted chalcones, and mixtures thereof; flavones selected from unsubstituted flavones, mono-substituted flavones, di-substituted flavones, and mixtures thereof; one or more isoflavones; coumarins selected from unsubstituted coumarins, mono-substituted coumarins, di-substituted coumarins, and mixtures thereof; chromones selected from unsubstituted chromones, mono-substituted chromones, di-substituted chromones, and mixtures thereof; one or more dicoumarols; one or more chromanones; one or more chromanols; isomers (e.g., cis/trans isomers) thereof; and mixtures thereof. By the term "substituted" as used herein means flavonoids wherein one or more hydrogen atom of the flavonoid has been independently replaced with hydroxyl, C1-C8 alkyl, C1-C4 alkoxyl, O-glycoside, and the like or a mixture of these substituents.

Examples of suitable flavonoids include, but are not limited to, unsubstituted flavanone, mono-hydroxy flavanones (e.g., 2'-hydroxy flavanone, 6-hydroxy flavanone, 7-hydroxy flavanone, etc.), mono-alkoxy flavanones (e.g., 5-methoxy flavanone, 6-methoxy flavanone, 7-methoxy flavanone, 4'-methoxy flavanone, etc.), unsubstituted chalcone (especially unsubstituted trans-chalcone), mono-hydroxy chalcones (e.g., 2'-hydroxy chalcone, 4'-hydroxy chalcone, etc.), di-hydroxy chalcones (e.g., 2',4-dihydroxy chalcone, 2',4'-dihydroxy chalcone, 2,2'-dihydroxy chalcone, 2',3-dihydroxy chalcone, 2',5'-dihydroxy chalcone, etc.), and tri-hydroxy chalcones (e.g., 2',3',4'-trihydroxy chalcone, 4,2',4'-trihydroxy chalcone, 2,2',4'-trihydroxy chalcone, etc.), unsubstituted flavone, 7,2'-dihydroxy flavone, 3',4'-dihydroxy naphthoflavone, 4'-hydroxy flavone, 5,6-benzoflavone, and 7,8-benzoflavone, unsubstituted isoflavone, daidzein (7,4'-dihydroxy isoflavone), 5,7-dihydroxy-4'-methoxy isoflavone, soy isoflavones (a mixture extracted from soy), unsubstituted coumarin, 4-hydroxy coumarin, 7-hydroxy coumarin, 6-hydroxy-4-methyl coumarin, unsubstituted chromone, 3-formyl chromone, 3-formyl-6-isopropyl chromone, unsubstituted dicoumarol, unsubstituted chromanone, unsubstituted chromanol, and mixtures thereof.

Among these flavanoid compounds, preferred are unsubstituted flavanone, methoxy flavanones, unsubstituted chalcone, 2',4-dihydroxy chalcone, isoflavone, flavone, and mixtures thereof, more preferably soy isoflavones.

Other non-limiting examples of flavanoid compounds suitable for use as skin benefit agents herein are described in U.S. Patents 5,686,082 and 5,686,367.

**vii. Anti-Inflammatory Agents**

The auxiliary skin benefit agent for use in the present composition can include anti-inflammatory agents, preferred concentrations of which range from 0.1% to 10%, more preferably from 0.5% to 5%, by weight of the composition.

Non-limiting examples of steroidal anti-inflammatory agents suitable for use herein include corticosteroids such as hydrocortisone, hydroxyltriamcinolone, alphamethyl dexamethasone, dexamethasone-phosphate, beclomethasone dipropionates, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fluosinolone acetonide, fluocinonide, flucortine butylesters, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone, fludrocortisone, diflurosone diacetate, fluradrenolone acetonide, medrysone, amcinafel, amcinafide, betamethasone and the balance of its esters, chloroprednisone, chlorprednisone acetate, clocortelone, clescinolone, dichlorisone, diflurprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate, triamcinolone, and mixtures thereof may be used. The preferred steroidal anti-inflammatory for use is hydrocortisone.

Nonsteroidal anti-inflammatory agents are also suitable for use herein as skin benefit agents in the active phase of the compositions. Non-limiting examples of nonsteroidal anti-inflammatory agents suitable for use herein include oxicams (e.g., piroxicam, isoxicam, tenoxicam, sudoxicam, CP-14,304); salicylates (e.g., aspirin, disalcid, benorylate, trilisate, safapryn, solprin, diflunisal, fendosal); acetic acid derivatives (e.g., diclofenac, fenclofenac, indomethacin, sulindac, tolmetin, isoxepac, furofenac, tiopinac, zidometacin, acematacin, fentiazac, zomepirac, clindanac, oxepinac, felbinac, ketorolac); fenamates (e.g., mefenamic, meclofenamic, flufenamic, niflumic, tolfenamic acids); propionic acid derivatives (e,g., ibuprofen, naproxen, benoxaprofen, flurbiprofen, ketoprofen, fenoprofen, fenbufen, indopropfen, pirprofen, carprofen, oxaprozin, pranoprofen, miroprofen, tioxaprofen, suprofen, alminoprofen, tiaprofenic); pyrazoles (e.g., phenylbutazone, oxyphenbutazone, feprazone, azapropazone, trimethazone); and combinations thereof as well as any dermatologically acceptable salts or esters of thereof.

Other non-limiting examples of suitable anti-inflammatory or similar other skin benefit agents include candelilla wax, bisabolol (e.g., alpha bisabolol), aloe vera, plant sterols (e.g., phytosterol), Manjistha (extracted from plants in the genus Rubia, particularly Rubia Cordifolia), and Guggal (extracted from plants in the genus Commiphora, particularly Commiphora Mukul), kola extract, chamomile, red clover extract, sea whip extract, and combinations thereof.

Other non-limiting examples of suitable anti-inflammatory or similar other skin benefit agents include compounds of the Licorice (the plant genus/species Glycyrrhiza glabra) family, including glycyrrhetic acid, glycyrrhizic acid, and derivatives thereof (e.g., salts and esters). Suitable salts of the foregoing compounds include metal and ammonium salts. Suitable esters include $C_2$ - $C_{24}$ saturated or unsaturated esters of the acids, preferably $C_{10}$- $C_{24}$, more preferably $C_{16}$- $C_{24}$. Specific non-limiting examples of the foregoing include oil soluble licorice extract, the glycyrrhizic and glycyrrhetic acids themselves, monoammonium glycyrrhizinate, monopotassium glycyrrhizinate, dipotassium glycyrrhizinate, 1-beta-glycyrrhetic acid, stearyl glycyrrhetinate, and 3-stearyloxy-glycyrrhetinic acid, disodium 3-succinyloxy-beta-glycyrrhetinate, and combinations thereof.

**viii. Anti-Cellulite Agents**

The auxiliary skin benefit agent for use in the compositions of the present invention anti-cellulite agents, non-limiting examples of which include xanthine compounds such as caffeine, theophylline, theobromine, aminophylline, and combinations thereof.

**ix. Topical Anesthetics**

The auxiliary skin benefit agent for use in the present invention include topical anesthetics, non-limiting examples of which include benzocaine, lidocaine, bupivacaine, chlorprocaine, dibucaine, etidocaine, mepivacaine, tetracaine, dyclonine, hexylcaine, procaine, ketamine, pramoxine, phenol, pharmaceutically acceptable salts thereof, and combinations thereof.

**x. Tanning Actives**

The auxiliary skin benefit agent for use in the present invention include tanning actives, preferred concentrations of which range from 0.1% to 20% by weight of the composition. Non-limiting examples of such tanning agents include dihydroxyacetone, which is also known as DHA or 1,3-dihydroxy-2-propanone.

### xi. Skin Lightening Agents

The auxiliary skin benefit agent for use in the present invention can include skin lightening agents, preferred concentrations of which range from 0.1% to 10%, more preferably from 0.2% to 5%, more preferably from 0.5% to 2%, by weight of the composition. Non-limiting examples of skin lightening agents suitable for use herein include kojic acid, arbutin, ascorbic acid and derivatives thereof (e.g., magnesium ascorbyl phosphate or sodium ascorbyl phosphate), and extracts (e.g., mulberry extract, placental extract) as well as titanium dioxide and zinc oxide. Non-limiting examples of skin lightening agents suitable for use herein also include those described in WO 95/34280, WO 95/07432, and WO 95/23780.

### xii. Skin Soothing and Skin Healing Actives

The auxiliary skin benefit agent for use in the present invention include skin soothing and skin healing actives, preferred concentrations of which range from 0.1% to 30%, more preferably from 0.5% to 20%, still more preferably from 0.5% to 10%, by weight of the composition. Non-limiting examples of skin soothing or skin healing actives suitable for use herein include panthenoic acid derivatives (e.g., panthenol, dexpanthenol, ethyl panthenol), aloe vera, allantoin, bisabolol, and dipotassium glycyrrhizinate.

### xiii. Antimicrobial Actives

The auxiliary skin benefit agent for use in compositions of the present invention may include antimicrobial actives, preferred concentrations of which range from 0.001% to 10%, more preferably from 0.01% to 5%, and still more preferably from 0.05% to 2%, by weight of the compositions.

Non-limiting examples of antimicrobial actives for use herein includes β-lactam drugs, quinolone drugs, ciprofloxacin, norfloxacin, tetracycline, erythromycin, amikacin, 2,4,4'-trichloro-2'-hydroxy diphenyl ether, 3,4,4'-trichlorobanilide, phenoxyethanol, phenoxy propanol, phenoxyisopropanol, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, ketaconazole, amanfadine hydrochloride, amanfadine sulfate, octopirox, parachlorometa xylenol, nystatin, tolnaftate, zinc pyrithione, clotrimazole, and combinations thereof.

### xiv. Sunscreen Actives

The auxiliary skin benefit agent for use in the present invention may comprise a sunscreen active, either organic or inorganic sunscreen actives. Among the inorganic sunscreens useful herein are metallic oxides such as titanium dioxide having an average primary particle size of from 15 nm to 100 nm, zinc oxide having an average primary particle size of from 15 nm to 150 nm, zirconium oxide having an average primary particle size of from 15 nm to 150 nm, iron oxide having an average primary particle size of from 15 nm to 500nm, and mixtures thereof.

The concentration of the sunscreen active for use in the composition preferably ranges from 0.1% to 20%, more typically from 0.5% to 10%, by weight of the composition. Exact amounts of such sunscreen actives will vary depending upon the sunscreen or sunscreens chosen and the desired Sun Protection Factor (SPF).

A wide variety of conventional organic sunscreen actives are also suitable for use herein, non-limiting examples of which include p-aminobenzoic acid, its salts and its derivatives (ethyl, isobutyl, glyceryl esters; p-dimethylaminobenzoic acid); anthranilates (i.e., o-amino-benzoates; methyl, menthyl, phenyl, benzyl, phenylethyl, linalyl, terpinyl, and cyclohexenyl esters); salicylates (amyl, phenyl, octyl, benzyl, menthyl, glyceryl, and di-pro-pyleneglycol esters); cinnamic acid derivatives (menthyl and benzyl esters, α-phenyl cinnamonitrile; butyl cinnamoyl pyruvate); dihydroxycinnamic acid derivatives (umbelliferone, methylumbelliferone, methylaceto-umbelliferone); trihydroxy-cinnamic acid derivatives (esculetin, methylesculetin, daphnetin, and the glucosides, esculin and daphnin); hydrocarbons (diphenylbutadiene, stilbene); dibenzalacetone and benzalacetophenone; naphtholsulfonates (sodium salts of 2-naphthol-3,6-disulfonic and of 2-naphthol-6,8-disulfonic acids); di-hydroxynaphthoic acid and its salts; o- and p-hydroxybiphenyldisulfonates; coumarin derivatives (7-hydroxy, 7-methyl, 3-phenyl); diazoles (2-acetyl-3-bromoindazole, phenyl benzoxazole, methyl naphthoxazole, various aryl benzothiazoles); quinine salts (bisulfate, sulfate, chloride, oleate, and tannate); quinoline derivatives

(8-hydroxyquinoline salts, 2-phenylquinoline); hydroxy- or methoxy-substituted benzophenones; uric and violuric acids; tannic acid and its derivatives (e.g., hexaethylether); (butyl carbotol) (6-propyl piperonyl) ether; hydroquinone; benzophenones (oxybenzene, sulisobenzone, dioxybenzone, benzoresorcinol, 2,2',4,4'-tetrahydroxybenzophenone, 2,2'-dihydroxy-4,4'-dimethoxybenzophenone, octabenzone; 4-isopropyldibenzoylmethane; butylmethoxydibenzoylmethane; etocrylene; octocrylene; [3-(4'-methylbenzylidene bornan-2-one), terephthalylidene dicamphor sulfonic acid and 4-isopropyl-dibenzoylmethane. Among these sunscreens, preferred are 2-ethylhexyl-p-methoxycinnamate (commercially available as PARSOL MCX), 4,4'-t-butyl methoxydibenzoyl-methane (commercially available as PARSOL 1789), 2-hydroxy-4-methoxybenzophenone, octyldimethyl-p-aminobenzoic acid, digalloyltrioleate, 2,2-dihydroxy-4-methoxybenzophenone, ethyl-4-(bis(hydroxy-propyl))aminobenzoate, 2-ethylhexyl-2-cyano-3,3-diphenylacrylate, 2-ethylhexyl-salicylate, glyceryl-p-aminobenzoate, 3,3,5-tri-methylcyclohexylsalicylate, methylanthranilate, p-dimethylaminobenzoic acid or aminobenzoate, 2-ethylhexyl-p-dimethyl-amino-benzoate, 2-phenylbenzimidazole-5-sulfonic acid, 2-(p-dimethylaminophenyl)-5-sulfonicbenzoxazoic acid, octocrylene and combinations thereof.

Non-limiting examples of other sunscreen actives suitable for use herein include those described in U.S. Patent No. 4,937,370 issued to Sabatelli on June 26, 1990, and U.S. Patent No. 4,999,186 issued to Sabatelli & Spirnak on March 12, 1991. Among those sunscreen actives described, preferred are 4-N,N-(2-ethylhexyl)methyl-aminobenzoic acid ester of 2,4-dihydroxybenzophenone; N,N-di-(2-ethylhexyl)-4-aminobenzoic acid ester with 4-hydroxydibenzoylmethane; 4-N,N-(2-ethylhexyl)methyl-aminobenzoic acid ester with 4-hydroxydibenzoylmethane; 4-N,N-(2-ethylhexyl) methyl-aminobenzoic acid ester of 2-hydroxy-4-(2-hydroxyethoxy)benzophenone; 4-N,N-(2-ethylhexyl)-methylaminobenzoic acid ester of 4-(2-hydroxyethoxy)dibenzoylmethane; N,N-di-(2-ethylhexyl)-4-aminobenzoic acid ester of 2-hydroxy-4-(2-hydroxyethoxy)benzophenone; and N,N-di-(2-ethylhexyl)-4-aminobenzoic acid ester of 4-(2-hydroxyethoxy)dibenzoylmethane and mixtures thereof. Especially preferred sunscreen actives include 4,4'-t-butylmethoxydibenzoylmethane, 2-ethylhexyl-p-methoxycinnamate, phenyl benzimidazole sulfonic acid, and octocrylene.

### xv. Visual Skin Enhancers

The auxiliary skin benefit agent for use in compositions of the present invention may include visual skin enhancement ingredients. These include ingredients that mask the appearance of any number of skin imperfections such as age spot, fine lines, wrinkles, blemishes etc., including but not limited to titanium dioxide, zinc oxide and iron oxides. Also suitable for use herein are organic particulates that diffuse light when deposited on the skin. Preferred concentrations of these ingredients range from 0.001% to 10%, more preferably from 0.01% to 5%, and still more preferably from 0.05% to 2%, by weight of the compositions.

### E. Aqueous Phase

The composition will preferably contain an aqueous phase that can comprise water and/or other hydroxyl containing solvents such as glycerin, propylene glycol and other water miscible solvents. The aqueous phase may be continuous or discontinuous depending on the formulation. The composition will contain at least 20% water even more preferably at least 30% and even more preferably 40% and most preferably at least 50%. The aqueous phase will comprise no more than 80% of the composition, even more preferably less than 75% of the composition, and most preferably less than 70% of the composition.

### F. Other Optional Ingredients

The CTFA Cosmetic Ingredient Handbook, Second Edition (1992) describes a wide variety of no limiting cosmetic and pharmaceutical ingredients commonly used in the personal care industry, which are suitable for use in the compositions of the present invention.

### i. Particles and Hydrophobically Modified Particles

The present invention may optionally comprise particles having a wide range of shapes, surface characteristics, and hardness characteristics which can be utilized to provide an optical effect and/or sensory effect. The particle size determines the opacity and luster. The particle size is determined by measuring the diameter thickness of the particulate material. The term "diameter" as used herein, means the largest distance across the major axis of the particulate material. Diameter can be determined by any suitable method known in the art, such as particle size analyzer Mastersizer 2000 manufactured by Malvern Instruments. The particles of the personal care compositions preferably have a diameter of at least 0.01 μm, more preferably at least 0.05 μm, even more preferably at least 0.1 μm, and still more preferably at least 0.2 μm. Particles such as interference pigments preferably have an average diameter not greater than 200 μm, more preferably not greater than 100 μm, even more preferably not greater than 80 μm, still more preferably not greater

than 60 μm. Other particles, such as polyoxyethlyene beads, preferably have an average diameter not greater than 1000 μm, more preferably not greater than 800 μm, and more preferably not greater than 600 μm.

The rinsable personal care composition of the present invention comprises from 0.01 to 20 weight percent of a particles. The particles of the personal care compositions preferably comprises no more than 20 weight percent of the composition, more preferably no more than 10 weight percent, even more preferably no more than 7 weight percent, and still more preferably no more than 5 weight percent of the personal care composition. The particles of the personal care composition preferably comprises at least 0.01 weight percent of the personal care composition, more preferably at least 0.05 weight percent, even more preferably at least 0.1 weight percent, and still more preferably at least 0.25 by weight of the composition.

These particles may be used as is, or may be hydrophobically modified (although some can in fact be 'naturally hydrophobic'). The particles can include but are not limited to those derived from a wide variety of materials including those derived from inorganic, organic, natural, and synthetic sources. Non-limiting examples of these materials include those selected from the group consisting of almond meal, alumina, aluminum oxide, titanium dioxide, mica, coated mica, sodium stearate, stearic acid, zinc stearate, aluminum silicate, apricot seed powder, aftapulgite, barley flour, bismuth oxychloride, boron nitride, calcium carbonate, calcium phosphate, calcium pyrophosphate, calcium sulfate, cellulose, chalk, chitin, clay, corn cob meal, corn cob powder, corn flour, corn meal, corn starch, diatomaceous earth, dicalcium phosphate, dicalcium phosphate dihydrate, fullers earth, hydrated silica, hydroxyapatite, iron oxide, jojoba seed powder, kaolin, loofah, magnesium trisilicate, mica, microcrystalline cellulose, montmorillonite, oat bran, oat flour, oatmeal, peach pit powder, pecan shell powder, polybutylene, polyethylene, polyisobutylene, polymethylstyrene, polypropylene, polystyrene, polyurethane, nylon, teflon (i.e. polytetrafluoroethylene), polyhalogenated olefins, pumice rice bran, rye flour, sericite, silica, silk, sodium bicarbonate, sodium silicoaluminate, soy flour synthetic hectorite, talc, tin oxide, titanium dioxide, tricalcium phosphate, walnut shell powder, wheat bran, wheat flour, wheat starch, zirconium silicate, and mixtures thereof. Also useful are micronized particles made from mixed polymers (e.g., copolymers, terpolymers, etc.), such as polyethylene/polypropylene copolymer, polyethylene/propylene/isobutylene copolymer, polyethylene/styrene copolymer, and the like.

In an embodiment of the present invention the particle is either naturally hydrophobic or has been hydrophobically modified. The hydrophobicity of a particle can be determined by the contact angle of a particle, such that the greater the contact angle, the greater the hydrophobicity of the particle. The particles of the present invention typically possess a contact angle of at least 60°, more preferably greater than 80°, even more preferably greater than 100°, still more preferably greater than 110°, even still more preferably greater than 120°, even still even more preferably greater than 130°, even still even more preferably greater than 135°. The hydrophobically modified particle (or "HMP") allows for the entrapment of the HMP within the dispersed phase and greater deposition of the HMP. In a preferred embodiment of the present invention, the invention contains both HMPs and a dispersed oil phase. Preferably the ratio of HMP to skin benefit agent is 1:1 to 1:100 more preferably 1:2 to 1:80, still more preferably 1:3 to 1:70 and most preferably 1:7 to 1:60.

The HMP of the present invention preferably has a hydrophobic coating comprising no more than 20 weight percent of the total particle weight, more preferably no more than 15 weight percent, even more preferably no more than 10 weight percent, even more preferably no more than 5 weight percent and even more preferably no more than 2 weight percent. The HMP of the present invention preferably has a hydrophobic coating comprising at least 0.1 weight percent of the total particle weight, more preferably at least 0.5 weight percent, even more preferably at least 1 weight percent. Non-limiting examples of the hydrophobic surface treatment useful herein include silicones, acrylate silicone copolymers, acrylate polymers, alkyl silane, isopropyl titanium triisostearate, sodium stearate, magnesium myristate, perfluoroalcohol phosphate, perfluoropolymethyl isopropyl ether, lecithin, carnauba wax, polyethylene, chitosan, lauroyl lysine, plant lipid extracts and mixtures thereof, preferably, silicones, silanes and stearates.

When formulated into a product, the HMP's are preferably entrapped within the skin benefit agent. This necessitates that the oil phase particle size is generally larger than the HMP. In a preferred embodiment of the invention, the skin benefit agent contains only a small number of HMPs per oil particles. Preferably this is less than 20, more preferably less than 10, most preferably less than 5. These parameters, the relative size of the oil droplets to the HMP and the approximate number of HMP particles per dispersed oil particles, can be determined by using visual inspection with light microscopy.

The HMP and skin benefit agent can be mixed into the composition via a premix or separately. For the case of separate addition, the hydrophobic pigments partition into the skin benefit agent or oil phase during the processing of the formulation. However, preferably the HMP may not be supplied dispersed in liquid prior to their incorporation into the personal care composition of the present invention.

The composition of the present invention can further comprise hydophobically modified particles selected from the group consisting of hydrophobically modified interference pigments, hydrophobically modified non-platelet particles and mixtures thereof. Preferred HMP particles include interference pigments, such as those disclosed in co-pending and commonly assigned under U.S. Patent Application Number 10/841,173 filed on May 7, 2004 by Clapp, et al titled "Personal Care Compositions Containing Hydrophobically Modified Interference Pigments," or may be non-platelet

particles, such as those disclosed in co-pending and commonly assigned under U.S. Patent Application Number 11/057,957, filed on February 15, 2005 by Taylor, et al titled "Personal Care Compositions Containing Hydrophobically Modified Non-Platelet Particles."

**ii. Thickeners/Aqueous Phase Stability Agent**

The compositions of the present invention, in some embodiments, may further include one or more thickeners/aqueous phase stability agents. Because different stability agents thicken with different efficiencies, it is difficult to provide an accurate compositional range, however, when present, the composition preferably comprises no more than 10 weight percent, more preferably no more than 8 weight percent, and still more preferably no more than 7 weight percent of the personal care composition. When present, the thickening/aqueous phase stability agent preferably comprises at least 0.01 weight percent, more preferably at least 0.05 weight percent, and still more preferably at least 0.1 weight percent of the personal care composition. It can often be useful to blend different Thickeners/Aqueous Phase Stability Agents together to generate an optimal stability and rheology profile.

Non-limiting examples of thickening agents useful herein include carboxylic acid polymers such as the carbomers (such as those commercially available under the tradename CARBOPOL® 900 series from B.F. Goodrich; e.g., CARBOPOL® 954) and the Luvigel series from BASF. Other suitable carboxylic acid polymeric agents include copolymers of $C_{10-30}$ alkyl acrylates with one or more monomers of acrylic acid, methacrylic acid, or one of their short chain (i.e., $C_{1-4}$ alcohol) esters, wherein the crosslinking agent is an allyl ether of sucrose or pentaerytritol. These copolymers are known as acrylates/$C_{10-30}$ alkyl acrylate crosspolymers and are commercially available as CARBOPOL® 1342, CARBOPOL® 1382, CARBOPOL Ultrez 21, PEMULEN TR-1, and PEMULEN TR-2, from B.F. Goodrich.

Other non-limiting examples of thickening agents include crosslinked polyacrylate polymers including both cationic and nonionic polymers.

Still other non-limiting examples of thickening agents include the polyacrylamide polymers, especially nonionic polyacrylamide polymers including substituted branched or unbranched polymers. More preferred among these polyacrylamide polymers is the nonionic polymer given the CTFA designation polyacrylamide and isoparaffin and laureth-7, available under the Tradename SEPIGEL 305 from Seppic Corporation (Fairfield, NJ). Other polyacrylamide polymers useful herein include multi-block copolymers of acrylamides and substituted acrylamides with acrylic acids and substituted acrylic acids. Commercially available examples of these multi-block copolymers include HYPAN SR150H, SS500V, SS500W, SSSA100H, from Lipo Chemicals, Inc., (Patterson, NJ).

Another non-limiting class of thickening agents useful herein are the polysaccharides. Non-limiting examples of polysaccharide gelling agents include those selected from cellulose, and cellulose derivatives. Preferred among the alkyl hydroxyalkyl cellulose ethers is the material given the CTFA designation cetyl hydroxyethylcellulose, which is the ether of cetyl alcohol and hydroxyethylcellulose, sold under the tradename NATROSEL® CS PLUS from Aqualon Corporation (Wilmington, DE). Other useful polysaccharides include scleroglucans which are a linear chain of (1-3) linked glucose units with a (1-6) linked glucose every three units, a commercially available example of which is CLEAROGEL™ CS11 from Michel Mercier Products Inc. (Mountainside, NJ).

Another non-limiting class of thickening agents useful herein are the gums. Non-limiting examples of gums useful herein include hectorite, hydrated silica, xantham gum, and mixtures thereof.

Yet another non-limiting class of thickening agents useful herein are the modified starches. Acrylate modified starches such as WATERLOCK® from Grain Processing Corporation may be used. Hydroxypropyl starch phosphate, tradename STRUCTURE XL from National Starch is another example of a useful modified starch, and other useful examples include ARISTOFLEX HMB (Ammonium Acrylodimethyltaruate/Beheneth-25 Methacrylate Crosspolymer) from Clariant and cationic stabylens.

Glycol fatty acid esters, such as ethylene glycol distearate may also be used as stabilizers.

**iii. Cationic Deposition Polymers**

The present invention may also contain cationic deposition polymer Concentrations of the cationic deposition polymer preferably range from 0.025% to 3%, more preferably from 0.05% to 2%, even more preferably from 0.1% to 1%, by weight of the personal care composition.

**iv. Silicones**

Silicones such as polydialkylsiloxanes, polydiarylsiloxanes, polyalkarylsiloxanes, and cylcomethicones having 3 to 9 silicon atoms can be useful optional ingredients in the present compositions. These silicones include both volatile and nonvolatile materials. These silicones are disclosed in U.S. Patent No. 5,069,897, to Orr, issued December 3, 1991, which is incorporated by reference herein in its entirety. The polyalkylsiloxanes include, for example, polyalkylsiloxanes

with viscosities of from 0.5 to 600,000 centistokes at 25°C. Such polyalkylsiloxanes correspond to the general chemical formula $R_3SiO[R_2SiO]_xSiR_3$ wherein R is an alkyl group (preferably R is methyl or ethyl, more preferably methyl) and x is an integer chosen to achieve the desired viscosity. Commercially available polyalkylsiloxanes include the polydimethylsiloxanes, which are also known as dimethicones, nonlimiting examples of which include the VICASIL® series sold by General Electric Company and the Dow Corning® 200 series sold by Dow Coming Corporation. Cyclic polyalkylsiloxanes useful herein include those corresponding to the general chemical formula $[SiR_2\text{-}O]_n$ wherein R is an alkyl group (preferably R is methyl or ethyl, more preferably methyl) and n is an integer from 3 to 9, more preferably n is an integer from 3 to 7, and most preferably n is an integer from 5 to 6. When R is methyl, these materials are typically referred to as cyclomethicones. Also useful are materials such as trimethylsiloxysilicate, which is a polymeric material corresponding to the general chemical formula $[(CH_2)_3SiO_{1/2}]_x[SiO_2]_y$, wherein x is an integer from 1 to 500 and y is an integer from 1 to 500. A commercially available trimethylsiloxysilicate is sold as a mixture with dimethicone as Dow Corning® 593 fluid. Also useful herein are dimethiconols, which are hydroxy terminated dimethyl silicones. These materials can be represented by the general chemical formulas $R_3SiO[R_2SiO]_xSiR_2OH$ and $HOR_2SiO[R_2SiO]_xSiR_2OH$ wherein R is an alkyl group (preferably R is methyl or ethyl, more preferably methyl). Commercially available dimethiconols are typically sold as mixtures with dimethicone or cyclomethicone (e.g. Dow Corning® 1401, 1402, and 1403 fluids). Also useful herein are polyalkylaryl siloxanes, with polymethylphenyl siloxanes having viscosities from 15 to 65 centistokes at 25°C being preferred. These materials are available, for example, as SF 1075 methylphenyl fluid (sold by General Electric Company) and 556 Cosmetic Grade phenyl trimethicone fluid (sold by Dow Corning Corporation). The silicones described herein can be blended with silicone gums to deliver desirable sensory and skin conditioning benefits. Such blends are commercially available from Dow Corning and GE Silicones. In addition, emulsified silicone gums are commercially available and suitable for use in the present compositions.

When present, the present compositions typically comprise from 0.1% to 25%, preferably from 0.5% to 20%, and more preferably from 1% to 10%, by weight of the composition, of silicone.

**v. Silicone Elastomers**

The compositions of the present invention can optionally further comprise from 0.1% to 30%, by weight of the composition, of a silicone elastomer component. Preferred levels of silicone elastomer are from 0.5% to 30%, and more preferably from 1% to 20%, by weight of the composition.

Suitable for use herein are silicone elastomers which can be emulsifying or non-emulsifying crosslinked siloxane elastomers or mixtures thereof. No specific restriction exists as to the type of curable organopolysiloxane composition that can serve as starting material for the crosslinked organopolysiloxane elastomer. Examples in this respect are addition reaction-curing organopolysiloxane compositions which cure under platinum metal catalysis by the addition reaction between SiH-containing diorganopolysiloxane and organopolysiloxane having silicon-bonded vinyl groups; condensation-curing organopolysiloxane compositions which cure in the presence of an organotin compound by a dehydrogenation reaction between hydroxyl-terminated diorganopolysiloxane and SiH-containing diorganopolysiloxane and condensation-curing organopolysiloxane compositions which cure in the presence of an organotin compound or a titanate ester.

Addition reaction-curing organopolysiloxane compositions can be preferred for their rapid curing rates and excellent uniformity of curing. A preferred addition reaction-curing organopolysiloxane composition is prepared from:

(A) an organopolysiloxane having at least 2 lower alkenyl groups in each molecule;
(B) an organopolysiloxane having at least 2 silicon-bonded hydrogen atoms in each molecule; and
(C) a platinum-type catalyst.

The compositions of the present invention can optionally include an emulsifying crosslinked organopolysiloxane elastomer, a non-emulsifying crosslinked organopolysiloxane elastomer, or a mixture thereof. The term "non-emulsifying," as used herein, defines crosslinked organopolysiloxane elastomers from which polyoxyalkylene units are absent. The term "emulsifying," as used herein, means crosslinked organopolysiloxane elastomers having at least one polyoxyalkylene (e.g., polyoxyethylene or polyoxypropylene) unit. Preferred emulsifying elastomers herein include polyoxyalkylene modified elastomers formed from divinyl compounds, particularly siloxane polymers with at least two free vinyl groups, reacting with Si-H linkages on a polysiloxane backbone. Preferably, the elastomers are dimethyl polysiloxanes crosslinked by Si-H sites on a molecularly spherical MQ resin. Emulsifying crosslinked organopolysiloxane elastomers can notably be chosen from the crosslinked polymers described in U.S. 5,412,004, U.S. 5,837,793, and U.S. 5,811,487. In addition, an emulsifying elastomer comprised of dimethicone copolyol crosspolymer (and) dimethicone is available from Shin Etsu under the tradename KSG-21.

Advantageously, the non-emulsifying elastomers are dimethicone/vinyl dimethicone crosspolymers. Such dimethicone/vinyl dimethicone crosspolymers are supplied by a variety of suppliers including Dow Coming (DC 9040 and DC 9041), General Electric (SFE 839), Shin Etsu (KSG-15, 16, 18 [dimethicone/phenyl vinyl dimethicone crosspolymer]),

and Grant Industries (GRANSIL™ line of elastomers). Cross-linked organopolysiloxane elastomers useful in the present invention and processes for making them are further described in U.S. 4,970,252, U.S. 5,760,116, and U.S. 5,654,362. Additional crosslinked organopolysiloxane elastomers useful in the present invention are disclosed in Japanese Patent Application JP 61-18708, assigned to Pola Kasei Kogyo KK.

Commercially-available silicone elastomers preferred for use herein are Dow Coming's 9040 silicone elastomer blend, Shin Etsu's KSG-21, and mixtures thereof.

**G. Analytical Methods**

**i. Lipid Rheology Test**

Lipid rheology is measured on a TA Instruments AR2000 stress-controlled rheometer with a Peltier temperature controlled sample stage or an equivalent. A parallel plate geometry is used with a 40mm plate and a 1mm gap. The lower plate is heated to 85°C and the melted lipid and structurant (if present) is added onto the lower plate and allowed to equilibrate. The upper plate is then lowered to the 1mm gap while ensuring the lipid fills the gap fully, [spinning the top plate and adding more lipid to promote wicking], and the sample is cooled quickly to 25°C and equilibrated at 25°C for 5 minutes. Viscosity is then measured using a stress-ramp procedure common on these types of machines using a logarithmic stress ramp from 20 to 2500Pa for a 2-minute duration, with 60 measurements points per decade. The starting and ending stress is sufficient to induce flow and reach a shear rate of at least 10 sec-1. Viscosity is recorded and the data fitted to a power law model using Equation 1. Only points between 0.001 sec-1 and 40 seconds-1 are to be used in the power law fit. The viscosity at 1.0 sec-1 is calculated from Equation 1. One should carefully watch the sample during the test so that when the material is ejected from under the plate, the method is stopped.

Equation 1:

$$\eta = \kappa \bullet \gamma(\mathrm{dot})^{(n-1)}$$

where η= viscosity, κ is the consistency and γ (dot) is the shear rate, and n is the shear index.

**ii. Lipid Modulus Test**

The Lipid Modulus test utilizes the data collected from the Lipid Rheology Test. In this case, the data is plotted as stress (Pa) vs. %Strain. The Modulus is obtained by recording the value at 200% strain (using visual extrapolation if there is not a data point at 200% strain) and dividing this value by 2 (200% strain is a strain of 2) to obtain the lipid modulus. In general 5 samples are run and the high and low values discarded and the middle three runs averaged to obtain a Lipid Modulus Value.

**iii. Particle Contact Angle Test**

The Particle Contact Angle Test is used to determine hydrophobicity of shiny particles. The greater the contact angle the greater the hydrophobicity of the particle.

A Spectra-Tech Qwik Handi-Press (Thermo Nicolet, Madison, WI) is used to compress the powder into 7-mm diameter discs. After applying firm hand pressure, the compression is held for 1 min prior to releasing pressure and removing the disc. The disc is examined for smoothness and rejected unless the surface is smooth. First Ten Angstrom FTÅ 200 (First Ten Angstrom, Portsmouth, VA) contact angle analyzer is employed to determine advancing and receding contact angles. 7 microliters of water (Millipore, Milli Q deionized, distilled) is dangled from the needle and slowly placed on the middle of the disc. The needle is left inserted in the drop but not in contact with disc. 0.1 microliters/second of water is pumped into the drop. Contact angle images were captured every 0.1 sec. until the maximum contact angle is obtained. The process is reversed to determine the receding contact angle in that the needle is left in the drop and fluid is removed at 0.1 microliters/second until the minimum contact angle is obtained. Images were obtained at 0.1 images/second, then calculate the contact angle. To calculate the contact angle, a curve is fitted to the profile of the drop on both sides of the drop. The baseline is drawn across the drop. The intersection of the curves and baseline is determined on both sides of the drop. The tangent (slopes) of the curve at the intersection is determined on both sides of the drop. The contact angle is the angle between the baseline and the tangent interior to the drop. The average contact angle is determined from the contact angles from both sides of the drop.

## H. Examples

The following examples further describe and demonstrate embodiments within the scope of the present invention. The examples are given solely for the purpose of illustration and are not to be construed as limitations of the present invention, as many variations thereof are possible without departing from the spirit and scope of the invention. All exemplified amounts are concentrations by weight of the total composition, i.e., wt/wt percentages, unless otherwise specified.

### a. Table 1:

| Rinsable personal cleansing compositions in Examples 1-4 can be manufactured according methods described below and using the ingredients in Table 1. | | | | |
|---|---|---|---|---|
| **Ingredient** | **Ex. 1** | **Ex.2** | **Ex. 3** | **Ex.4** |
| **I. Aqueous Phase Composition** | wt% | wt% | wt% | wt% |
| **Polymer** | | | | |
| Structure XL (Hydroxypropyl Starch Phosphate from National Starch) | | | | |
| Pemulen TR2 (Acrylates/C10-30 | | 0.3 | | |
| Alkyl Acrylate Crosspolymer from Noveon, Inc.) | | | | |
| Carbopol Ultrez 10 (Carbomer from Noveon, Inc.) | | | 1.8 | |
| Xanthan Gum | | | 0.1 | |
| Sepigel 305 (Polyacrylamide and C13-14 Isoparaffin and Laureth-7 from Seppic) | 3.0 | | | 2.7 |
| **Surfactant** | | | | |
| Polawax (Emulsifying Wax NF from Croda) | | | | 2.5 |
| PEG-100 Stearate | | | | |
| Tween 80 (Polysorbate 80 from Uniqema Americas) | 2.1 | 0.25 | 0.3 | |
| Ammonium Lauryl Sulfate | | | | |

(continued)

| Rinsable personal cleansing compositions in Examples 1-4 can be manufactured according methods described below and using the ingredients in Table 1. | | | | |
|---|---|---|---|---|
| **Ingredient** | **Ex. 1** | **Ex.2** | **Ex. 3** | **Ex.4** |
| **I. Aqueous Phase Composition** | wt% | wt% | wt% | wt% |
| Incroquat Behenyl TMS (Behenetrimonium methosulfate and cetearyl alcohole from Croda) | | | | |
| **Additional Ingredients** | | | | |
| AMP-95 (Amino Methyl Propanol from Angus Chemical) | | 0.14 | 0.1 | |
| Glycerine | 0.6 | 0.5 | | 0.7 |
| Salicylic Acid | | | | |
| Fragrance | 1.0 | 1.0 | 1.0 | 1.0 |
| Preservatives | 0.8 | 0.8 | 0.8 | 0.8 |
| Water | Q.S. | Q.S. | Q.S. | Q.S. |
| **II. High Modulus Lipid Phase** | | | | |
| Superwhite Protopet (Petrolatum from WITCO) | 20 | 20 | | |
| Hydrobrite 1000 PO (Mineral Oil from WITCO) | | | | |
| G-2180 Petrolatum from WITCO | | | 20 | 10 |
| W-445 Microcrystalline Wax | | 5 | | |
| Jeenate 4H Polyethylene from Jeen, Inc. | 3 | | 2 | |
| Soybean Oil | | | | |
| Puresyn 101LT (Polydecene from Exxon Mobile) | | | 1 | |
| Lipovol Sun (Sunflower Seed Oil from Lipo) | 2 | | | |
| **III Ester** | | | | |

(continued)

| Rinsable personal cleansing compositions in Examples 1-4 can be manufactured according methods described below and using the ingredients in Table 1. | | | | |
|---|---|---|---|---|
| **Ingredient** | **Ex. 1** | **Ex.2** | **Ex. 3** | **Ex.4** |
| **I. Aqueous Phase Composition** | wt% | wt% | wt% | wt% |
| Diisopropyl Sebacate | | | | |
| Ispopropyl Palmitate | | 5 | | 15 |
| Isopropyl Isostearate | 3 | | 2 | |

**b. Table 2:**

Rinsable personal cleansing compositions in Examples 5-7 can be manufactured according methods described below and using the ingredients in Table 2.

**Examples 7-12:**

| **Ingredient** | **Ex5** | **Ex. 6** | **Ex.7** |
|---|---|---|---|
| **I. Aqueous Phase Composition** | wt% | wt% | wt% |
| **Polymer** | | | |
| Structure XL (Hydroxypropyl Starch Phosphate from National Starch) | 3.5 | 3.5 | 4 |
| Pemulen TR2 (Acrylates/C10-30 Alkyl Acrylate Crosspolymer from Noveon, Inc.) | | | |
| Carbopol Ultrez 10 (Carbomer from Noveon, Inc.) | | | |
| Xanthan Gum | | | |
| Sepigel 305 (Polyacrylamide and C13-14 Isoparaffin and Laureth- 7 from Seppic) | | | |
| **Surfactant** | | | |
| Polawax (Emulsifying Wax NF from Croda) | 2.0 | 2.0 | 1 |
| PEG-100 Stearate | | | |
| Tween 80 (Polysorbate 80 from Uniqema Americas) | | | |
| Ammonium Lauryl Sulfate | | | 2.5 |
| Incroquat Behenyl TMS (Behenetrimonium methosulfate and cetearyl alcohole from Croda) | | | |
| **Additional Ingredients** | | | |
| AMP-95 (Amino Methyl Propanol from Angus Chemical) | | | |
| Glycerine | | | 0.5 |
| Salicylic Acid | | | |
| Fragrance | 1.0 | 1.0 | 1.0 |
| Preservatives | 0.8 | 0.8 | 0.8 |
| Water | Q.S. | Q.S. | Q.S. |
| **II. High Modulus Lipid Phase** | | | |

(continued)

| Ingredient | Ex5 | Ex. 6 | Ex.7 |
|---|---|---|---|
| Superwhite Protopet (Petrolatum from WITCO) | | | 10 |
| Hydrobrite 1000 PO (Mineral Oil from WITCO) | | | |
| G-2180 Petrolatum from WITCO | 15 | 22 | |
| W-445 Microcrystalline Wax | 3 | | |
| Jeenate 4H Polyethylene from Jeen, Inc. | | | |
| Soybean Oil | | | |
| Puresyn 101LT (Polydecene from Exxon Mobile) | | | |
| Lipovol Sun (Sunflower Seed Oil from Lipo) | 5 | | |
| **III Ester** | | | |
| Diisopropyl Sebacate | 2.5 | 3 | |
| Ispopropyl Palmitate | | 1 | 20 |
| Isopropyl Isostearate | | | |

**c. Methods for making the compositions in Table 1 and Table 2:**

The above Examples 1-7 can be manufactured via a variety of methods well known to those skilled in the art.

**i. Method 1**

In the first method, the High Modulus Lipid components are added and heated to 80°C to produce a clear mixture. The polymer and surfactant are then added to the lipid phase and mixed until well dispersed. 80°C water is then added to the oil phase and the polymer neutralized (as required in examples 2-3). The batch is then brought to a room temperature water bath to cool, and the additional ingredients are then added. Once the batch cools below 40°C, the ester is then added to the product. The product is mixed until smooth and then packaged at room temperature. In this method, the ester is effectively added separately from the high modulus lipid.

**ii. Method 2**

The procedure of Method 1 is followed with the exception that the ester is added to the product upon removal from heat and placing into the room temperature water bath. In this method, the ester is added separately from the high modulus lipid.

**iii. Method 3**

In this method, the water is placed into a container and heated to 80°C, polymer and surfactants are added and the High Modulus Lipid components are heated and mixed to clarity separately and then added to the batch. The batch is then removed from heat and placed in a room temperature water bath. The additional ingredients are then added, and upon reaching 40°C, ester is added to the batch. For examples 2-3 the polymer would then be neutralized. The product is mixed until smooth and then packaged at room temperature. In this method, the ester is added separately from the high modulus lipid.

**iv. Method 4**

The procedure of Method 3 is followed with the exception that the ester is added to the product upon removal from heat and placing into the room temperature water bath. In this method, the ester is added separately from the high modulus lipid.

**v. Method 5**

The procedure of Method 1 is followed, with the exception that the ester is added first, and the premixed and melted High Modulus Lipid components are added after the batch has reached 40°C. In this the method, the ester is added separately from the high modulus lipid.

**vi. Method 6**

The procedure of Method 3 is followed, with the exception that the ester is added to the hot aqueous phase, and the premixed and melted High Modulus Lipid phase is added after the batch has reached 40°C. In this method, the ester is added separately from the high modulus lipid.

**vii. Method 7**

The procedure of Method 1 is followed, with the exception that the ester is added to the hot high modulus lipid phase prior to the addition of any other raw materials. In this method, the ester and the high modulus lipid are premixed prior to addition to the product.

**viii. Method 8**

The procedure of method 3 is followed, with the exception that the high modulus lipid components are premixed with the ester (with heat to clarity) prior to addition to the batch at 80°C (batch temperature)

**ix. Method 9**

The procedure of Method 3 is followed, with the exception that the high modulus lipid components are premixed with the ester (with heat to clarity) prior to addition to the batch at 40°C (batch temperature).

**d. Table 3:**

Skin care compositions in the form of cleansing creams and cleansing milks may also be prepared by the following procedure described below and using the ingredients in Table 3.

| **Ingredients** | 13 | 14 | 15 |
|---|---|---|---|
| **Phase A** | | | |
| Water | QS 100 | QS 100 | QS 100 |
| Glycerin | 3 | 5 | 3 |
| Decyl Glucoside[1] | | 1.25 | 2.5 |
| Disodium EDTA | 0.01 | 0.01 | 0.01 |
| **Phase B** | | | |
| Isopropyl Palmitate[2] | 15 | 15 | 15 |
| Mineral Oil[3] | 1.75 | 1.75 | 1.75 |
| Petrolatum | 3.25 | 3.25 | 3.25 |
| Behenyl Alcohol | | | 0.25 |
| Stearyl Alcohol | 1.33 | 1.5 | .75 |
| Cetyl Alcohol | 1.33 | 1.5 | 2.5 |
| Distearyl Dimethyl Ammonium Chloride[4] | 1.0 | 1.25 | 1.5 |
| Steareth-21[5] | 0.5 | 0.5 | 0.5 |
| Steareth-2[6] | 0.25 | 0.25 | 0.25 |
| **Phase C** | | | |

(continued)

| Ingredients | 13 | 14 | 15 |
|---|---|---|---|
| Oxidized Polyethylene Beads[7] | | | 1.0 |
| Fragrance | 0.2 | 0.15 | 0.15 |
| Menthol | | | |
| **Phase D** | | | |
| Cetyl Betaine[8] | 2 | 2 | 2 |
| Sodium Lauryl Sulfate[9] | 1 | 1 | 1 |
| Glydant Plus | 0.3 | 0.3 | 0.3 |

[1] Available as Plantaren 2000USP (Cognis Corp)
[2] Available as Stepan IPP (Stepan)
[3] Available as Hydrobright 1000 (Crompton)
[4] Available as Arosurf TA-100 (Degussa)
[5] Available as Brij 721 (Uniqema)
[6] Available as Brij 72 Uniqema)
[7] Available as A-C Oxidized Polyethylene (Honeywell)
[8] Available as Lonzaine SP16 (Lonza)
[9] Available as Stepanal WAC (Stepan)

**e. Method for making the compositions in Table 3:**

The above Examples 13-15 can be manufactured via a variety of methods well known to those skilled in the art.

**i. Method 1**

In a suitable vessel, heat the Phase A ingredients with stirring to about 75°C. In a separate vessel, heat the Phase B ingredients with stirring to about 75°C. Add Phase B to Phase A with mixing. Add Phase C to Phases A&B. Cool the mixture to about 35°C. In a separate vessel, combine the Phase D ingredients and add to the remaining mixture with stirring.

**f. Table 4:**

Skin care compositions in the form of cleansing creams and cleansing milks may also be prepared by the following method described below and using the ingredients in Table 4.

| Ingredient | 17 |
|---|---|
| **Phase A:** | |
| **Water** | QS |
| Disodium EDTA | 0.02 |
| Glycerin | 5.0 |
| Sorbitol | 5.0 |
| Acrylates/C10-30 alkyl acrylate crosspolymer[1] | 0.25 |
| Luvigel EM[2] | |
| Hydroxypropyl Starch Phosphate[3] | 0.2 |
| Decyl Glucoside[4] | 2.5 |
| **Phase B:** | |
| Isopropyl Palmitate[5] | 15 |

(continued)

| Ingredient | 17 |
|---|---|
| **Phase A:** | |
| **Water** | QS |
| Mineral Oil[6] | 1.5 |
| Petrolatum | 3.5 |
| Steareth-21[7] | 0.5 |
| Steareth-2[8] | 0.25 |
| Cetearyl Alcohol / Polysorbate 60[9] | 5.0 |
| **Phase C:** | |
| DMDM Hydantoin / Iodopropynyl Butylcarbamate[10] | 0.30 |
| Fragrance | 0.2 |
| [1] Carbopol Ultrez-21 (Noveon)<br>2 Structure XL (National Starch)<br>3 Plantaren 2000 USP (Cognis)<br>4 Stepan IPP (Stepan)<br>5 Hydrobright 1000 (Crompton)<br>6 BRIJ™ 721 (Uniquema Corp.)<br>7 BRIJ™ 72 (Uniquema Corp.)<br>8 Lipowax (Lipo Chemicals)<br>9 Glydant Plus (Lonza) | |

**g. Method for making the compositions in Table 4:**

The above Example 17 can be manufactured via a variety of methods well known to those skilled in the art.

i. Method 1

Add Phase A ingredients into a suitable container. While mixing with a propeller, heat this mixture to 60 -70°C. Add Phase B to a suitable container. While mixing, heat this mixture to 60 - 75°C. Add phase B to phase A while mixing with a propeller until it is homogeneous. Mill the mixture of A and B for 1 - 3 minutes using a Tekmar mill at 9000 -15,000 rpm. Remove the Tekmar mill and replace with a propeller mixer. Remove heat and continue mixing phase A/B until temperature drops to 45 - 50°C, add phase C and continue mixing with a propeller. While mixing with a propeller, cool the batch to 35 - 40°C, ensuring that the batch is smooth and homogeneous. Adjust pH to approximately 5.5 - 7 using a citric acid or sodium hydroxide solution. Transfer the batch to containers for storage.

## Claims

**1.** A rinsable personal care composition comprising:

(a) from 0.1% to 50%, by weight of said composition, of surfactant of which from 0.01 to 4% by weight of the composition is non-ionic surfactant;
(b) from 18% to 70%, by weight of said composition, of a skin benefit agent; said skin benefit agent comprising:

a high modulus lipid, having a lipid modulus of from 50 to 5000 Pa; and
an ester, said ester having less than 30 carbon atoms,

wherein the ratio by weight of high modulus lipid to ester is in the range from 30:1 to 1:15; and
(c) at least 20% by weight of said composition, of water but wherein the total amount of water and other hydroxyl containing solvents in the composition is less than 80% by weight of the composition.

2. The composition of claim 1, wherein said composition comprises no more than about 5%, by weight of said composition, of said surfactant.

3. The composition of claim 1 or claim 2, wherein said surfactant is in the form of a nonionic gel network.

4. The composition of Claim 1, wherein said high modulus lipid comprises an oil selected from the group consisting of hydrocarbon oils, triglycerides, polyesters, silicone oils, and mixtures thereof.

5. The composition of any one of the preceding claims, wherein a ratio of said high modulus lipid to said ester is from 1:10 to 30:1.

6. The composition of any one of the preceding claims, wherein said high modulus lipid has a lipid modulus of from 100 Pa to 3000 Pa.

7. The composition of any one of the preceding claims, wherein said ester comprises no more than 23 carbon atoms.

8. The composition of any one of the preceding claims, wherein said ester is selected from the group consisting of a di-ester, a tri-ester, a tetra-ester, a branched ester, a dimer, and mixtures thereof.

9. The composition according to any one of claims 1 to 8, comprising no more than 50%, by weight of the composition, of said skin benefit agent.

10. The composition according to any one of claims 1 to 9, wherein said composition comprises at least 30%, preferably at least 40% and more preferably at least 50%, by weight of the composition, of water.

11. The composition of any one of the preceding claims, wherein said composition further comprises a structurant, a thickener/aqueous phase stability agent, a hydrophobically modified particle, or mixtures thereof.

12. The composition of any one of the preceding claims, wherein said ester is added separately from said high modulus lipid during a process of making said composition.

13. The composition of any one of the preceding claims, wherein said ester is premixed witch said high modulus lipid during a process of making said composition.

14. A method for moisturizing skin, said method comprising the steps of:

cleansing said skin;
applying to said skin a composition according to any one of the preceding claims; and
rinsing said composition from said skin.

15. A method for removing make-up, said method comprising the steps of:

applying to said skin a composition according to any one of Claims 1 to 13; and,
rinsing or wiping away said composition from said skin.

**Patentansprüche**

1. Abspülbare Körperpflegezusammensetzung, umfassend:

(a) zu 0,1 Gew.-% bis 50 Gew.-% der Zusammensetzung Tensid, von dem 0,01 bis 4 Gew.-% der Zusammensetzung nichtionisches Tensid sind,
(b) zu 18 Gew.-% bis 70 Gew.-% der Zusammensetzung einen hautpflegenden Wirkstoff, wobei der hautpflegende Wirkstoff Folgendes umfasst:

ein hochmodules Lipid mit einem Lipidmodul von 50 bis 5000 Pa und
einen Ester, wobei der Ester weniger als 30 Kohlenstoffatome besitzt,

wobei das Gewichtsverhältnis von hochmodulem Lipid zu Ester im Bereich von 30:1 bis 1:15 liegt, und
(c) zu mindestens 20 Gew.-% der Zusammensetzung Wasser, wobei jedoch die Gesamtmenge an Wasser und anderen hydroxylhaltigen Lösemitteln in der Zusammensetzung weniger als 80 Gew.-% der Zusammensetzung beträgt.

**2.** Zusammensetzung nach Anspruch 1, wobei die Zusammensetzung zu nicht mehr als etwa 5 Gew.-% der Zusammensetzung das Tensid umfasst.

**3.** Zusammensetzung nach Anspruch 1 oder 2, wobei das Tensid in Form eines nichtionischen Gelnetzwerks vorliegt.

**4.** Zusammensetzung nach Anspruch 1, wobei das hochmodule Lipid ein Öl umfasst, ausgewählt aus der Gruppe bestehend aus Kohlenwasserstoffölen, Triglyceriden, Polyestern, Silikonölen und Mischungen davon.

**5.** Zusammensetzung nach einem der vorstehenden Ansprüche, wobei ein Verhältnis von dem hochmodulen Lipid zu dem Ester 1:10 bis 30:1 beträgt.

**6.** Zusammensetzung nach einem der vorstehenden Ansprüche, wobei das hochmodule Lipid einen Lipidmodul von 100 Pa bis 3000 Pa aufweist.

**7.** Zusammensetzung nach einem der vorstehenden Ansprüche, wobei der Ester nicht mehr als 23 Kohlenstoffatome umfasst.

**8.** Zusammensetzung nach einem der vorstehenden Ansprüche, wobei der Ester ausgewählt ist aus der Gruppe bestehend aus einem Diester, einem Triester, einem Tetraester, einem verzweigten Ester, einem Dimer und Mischungen davon.

**9.** Zusammensetzung nach einem der Ansprüche 1 bis 8, umfassend zu nicht mehr als 50 Gew.-% der Zusammensetzung den hautpflegenden Wirkstoff.

**10.** Zusammensetzung nach einem der Ansprüche 1 bis 9, wobei die Zusammensetzung zu mindestens 30 Gew.-%, vorzugsweise mindestens 40 Gew.-% und bevorzugter mindestens 50 Gew.-% der Zusammensetzung Wasser umfasst.

**11.** Zusammensetzung nach einem der vorstehenden Ansprüche, wobei die Zusammensetzung ferner ein Strukturmittel, ein Verdickungsmittel/Stabilisierungsmittel für wässrige Phasen, ein hydrophob modifiziertes Teilchen oder Mischungen davon umfasst.

**12.** Zusammensetzung nach einem der vorstehenden Ansprüche, wobei der Ester während eines Verfahrens zur Herstellung der Zusammensetzung getrennt von dem hochmodulen Lipid hinzugegeben wird.

**13.** Zusammensetzung nach einem der vorstehenden Ansprüche, wobei der Ester während eines Verfahrens zur Herstellung der Zusammensetzung mit dem hochmodulen Lipid vorgemischt wird.

**14.** Verfahren zur Hautbefeuchtung, wobei das Verfahren die folgenden Schritte umfasst:

Reinigen der Haut,
Auftragen einer Zusammensetzung nach einem der vorstehenden Ansprüche auf die Haut und
Abspülen der Zusammensetzung von der Haut.

**15.** Verfahren zum Entfernen von Make-up, wobei das Verfahren die folgenden Schritte umfasst:

Auftragen einer Zusammensetzung nach einem der Ansprüche 1 bis 13 auf die Haut und
Abspülen oder Abwischen der Zusammensetzung von der Haut.

## Revendications

**1.** Composition de soin personnel rinçable comprenant :

(a) de 0,1 % à 50 % en poids de ladite composition, d'un agent tensioactif duquel de 0,01 à 4 % en poids de la composition est un agent tensioactif non ionique ;
(b) de 18 % à 70 % en poids de ladite composition, d'un agent de soin cutané ; ledit agent de soin cutané comprenant :

un liquide à module élevé, ayant un module de lipide allant de 50 à 5000 Pa ; et
un ester, ledit ester ayant moins de 30 atomes de carbone,

dans laquelle le rapport en poids du lipide à module élevé sur l'ester est dans la gamme de 30:1 à 1:15; et
(c) au moins 20 % en poids de ladite composition, d'eau, mais dans laquelle la quantité totale d'eau et d'autres solvants à contenu hydroxyle dans la composition est moins de 80 % en poids de la composition.

**2.** Composition selon la revendication 1, où ladite composition ne comprend pas plus d'environ 5 % en poids de ladite composition, dudit agent tensioactif.

**3.** Composition selon la revendication 1 ou la revendication 2, dans laquelle ledit agent tensioactif est sous la forme d'un réseau de gel non ionique.

**4.** Composition selon la revendication 1, dans laquelle ledit lipide à module élevé comprend une huile choisie dans le groupe constitué d'huiles hydrocarbure, triglycérides, polyesters, huiles de silicone, et leurs mélanges.

**5.** Composition selon l'une quelconque des revendications précédentes, dans laquelle un rapport dudit lipide à module élevé sur ledit ester va de 1:10 à 30:1.

**6.** Composition selon l'une quelconque des revendications précédentes, dans laquelle ledit lipide à module élevé a un module de lipide allant de 100 Pa à 3000 Pa.

**7.** Composition selon l'une quelconque des revendications précédentes, dans laquelle ledit ester ne comprend pas plus de 23 atomes de carbone.

**8.** Composition selon l'une quelconque des revendications précédentes, dans laquelle ledit ester est choisi dans le groupe constitué d'un di-ester, un tri-ester, un tétra-ester, un ester ramifié, un dimère, et leurs mélanges.

**9.** Composition selon l'une quelconque des revendications 1 à 8, ne comprenant pas plus de 50 % en poids de la composition, dudit agent de soin cutané.

**10.** Composition selon l'une quelconque des revendications 1 à 9, où ladite composition comprend au moins 30 %, de préférence au moins 40 % et plus préférablement au moins 50 % en poids de la composition, d'eau.

**11.** Composition selon l'une quelconque des revendications précédentes, où ladite composition comprend en outre un structurant, un épaississant/agent stabilisant de phase aqueuse, une particule rendue hydrophobe, ou leurs mélanges.

**12.** Composition selon l'une quelconque des revendications précédentes, dans laquelle ledit ester est ajouté séparément dudit lipide à module élevé durant un procédé de fabrication de ladite composition.

**13.** Composition selon l'une quelconque des revendications précédentes, dans laquelle ledit ester est prémélangé avec ledit lipide à module élevé durant un procédé de fabrication de ladite composition.

**14.** Procédé pour hydrater la peau, ledit procédé comprenant les étapes consistant à :

nettoyer ladite peau ;
appliquer sur ladite peau une composition selon l'une quelconque des revendications précédentes ; et rincer ladite composition de ladite peau.

**15.** Procédé pour retirer un maquillage, ledit procédé comprenant les étapes consistant à:

appliquer sur ladite peau une composition selon l'une quelconque des revendications 1 à 13 ; et,

rincer ou essuyer ladite composition de ladite peau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040223929 A1
- US 05795705 A, Taylor
- US 6280757 B, McAtee
- US 3929678 A, Laughlin
- US 4557853 A
- GB 809060 A, Thomas Hedley & Co., Ltd.
- US 2965576 A, E. R. Wilson
- US 2703798 A, A. M. Schwartz
- US 1985424 A, Piggott
- US 5681852 A, Bissett
- US 5652228 A, Bissett
- US 5607980 A, McAtee
- US 6217888 B, Oblong
- US 5487884 A, Bissett
- US 9116035 B, Bush
- US 9116034 B, Bush
- US 5686082 A
- US 5686367 A
- WO 9534280 A
- WO 9507432 A
- WO 9523780 A
- US 4937370 A, Sabatelli
- US 4999186 A, Sabatelli & Spirnak
- US 84117304 A, Clapp
- US 5069897 A, Orr
- US 5412004 A
- US 5837793 A
- US 5811487 A
- US 4970252 A
- US 5760116 A
- US 5654362 A
- JP 61018708 A


### Non-patent literature cited in the description


- McCutcheon's, Detergents and Emulsifiers. Publishing Corporation, 1986
- McCutcheon's, Functional Materials. 1992
- CTFA Cosmetic Ingredient Handbook. 1992